(19) Europäisches Patentamt
European Patent Office
Office européen des brevets

(11) Numéro de publication : **0 531 187 A1**

(12) **DEMANDE DE BREVET EUROPEEN**

(21) Numéro de dépôt : **92402301.3**

(22) Date de dépôt : **19.08.92**

(51) Int. Cl.[5] : **C12N 15/81,** C12N 1/19, C12P 21/02, // C07K15/00 , (C12N1/19, C12R1:465)

The microorganism(s) has (have) been deposited with Centraalbureau voor Schimmelculturen under number(s) CBS 289.92

(30) Priorité : **21.08.91 FR 9110476**

(43) Date de publication de la demande : **10.03.93 Bulletin 93/10**

(84) Etats contractants désignés : **PT**

(71) Demandeur : **RHONE-POULENC RORER SA**
**20, avenue Raymond Aron**
**F-92160 Antony (FR)**

(72) Inventeur : **Fleer, Reinhard**
**47 avenue Beauséjour**
**F-91440 Bures Sur Yvette (FR)**
Inventeur : **Saliola, Michele**
**Via Belle Azalee 13**
**I-00172 Rome (IT)**
Inventeur : **Les autres inventeurs ont renoncé à leur désignation**

(74) Mandataire : **Savina, Jacques et al**
**Rhône-Poulenc Rorer S.A. Direction Brevets**
**(t144) 20 avenue Raymond Aron**
**F-92165 Antony Cédex (FR)**

(54) **Promoteur de levure et son utilisation.**

(57) L'invention concerne des séquences d'ADN comprenant tout ou partie du promoteur du gène ADH4 de K.lactis ou d'un dérivé de celui-ci, et possédant une activité de promoteur transcriptionnel. Elle concerne également l'utilisation de ces séquences pour l'expression de gènes recombinés.

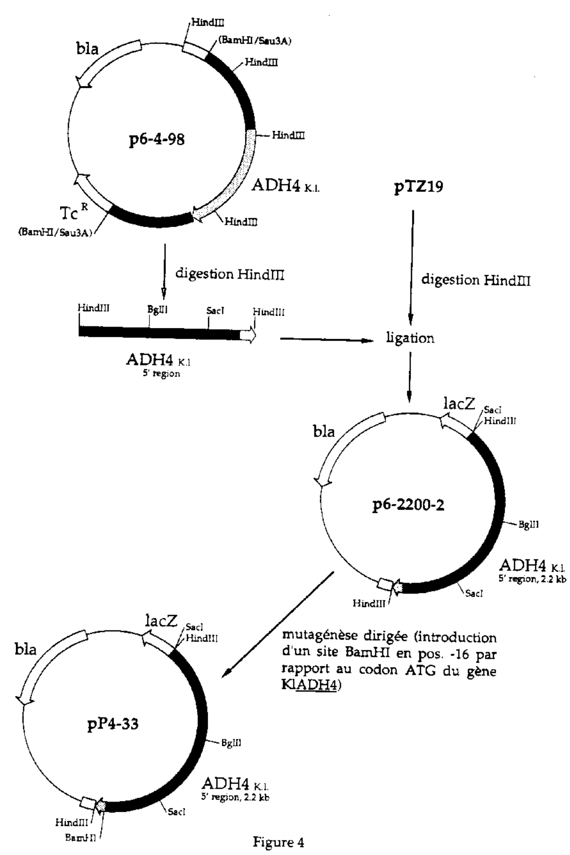

Figure 4

EP 0 531 187 A1

La présente invention concerne le domaine de la biologie moléculaire. Plus particulièrement, elle concerne une nouvelle séquence d'ADN présentant une activité de promoteur transcriptionnel, des vecteurs d'expression contenant cette séquence, et son utilisation pour la production de protéines recombinantes, et par exemple de protéines hétérologues. L'invention concerne aussi les cellules recombinées contenant cette séquence d'ADN.

Les progrès accomplis dans le domaine de la biologie moléculaire ont permis de modifier des microorganismes pour leur faire produire des protéines hétérologues. En particulier, de nombreuses études génétiques ont porté sur la bactérie E.coli. Toutefois, l'application industrielle de ces nouveaux modes de production est encore limitée, en particulier par les problèmes d'efficacité d'expression des gènes dans ces microganismes recombinés. Aussi, dans le but d'augmenter les performances de ces systèmes de production, des recherches ont été effectuées afin d'isoler des promoteurs forts, permettant d'obtenir des niveaux élevés d'expression de protéines hétérologues. Chez E.coli, on peut citer en particulier les promoteurs des opérons tryptophane et lactose.

Plus récemment, chez la levure S.cerevisiae, des études ont porté sur des promoteurs dérivés de gènes impliqués dans la glycolyse. On peut citer notamment les travaux sur le promoteur du gène de la 3-phosphoglycérate kinase PGK (Dobson et al., Nucleic Acid Res. 10, 1982, 2625 ; Hitzeman et al., Nucleic Acid Research 1982, 7791 ), sur celui du gène de la glyceraldéhyde-3-phosphate deshydrogénase GAPDH (Holland et al., J. Biol. Chem. 254, 1979, 9839 ; Musti et al., Gene 25, 1983, 133), sur celui du gène de l'alcool deshydrogénase 1 ADH1 (Bennentzen et al., J. Biol. Chem. 257, 1982, 3018 ; Denis et al., J.Biol.Chem. 25, 1983, 1165), ou sur celui du gène de l'enolase 1 ENO1. (Uemura et al., Gene 45, 1986, 65).

Récemment, des outils génétiques ont été développés afin de se servir de la levure Kluyveromyces comme cellule hôte pour la production de protéines recombinantes. La mise en évidence d'un plasmide de type 2-micron originaire de K. drosophilarum (plasmide pKD1 - EP 241 435) a permis d'établir un système hôte/vecteur très efficace pour la production de protéines recombinantes (EP 361 991). Cependant, les promoteurs utilisés dans ce système n'ont jamais été optimisés. En particulier, il s'agit essentiellement de promoteurs hétérologues, c'est-à-dire provenant d'autres microorganismes, tel que notamment S.cerevisiae. Cette situation peut engendrer différents inconvénients, et notamment limiter l'activité du promoteur à cause de l'absence de certains éléments de la machinerie transcriptionnelle (par exemple de trans-activateurs), présenter une certaine toxicité pour la cellule hôte due à une absence de régulation, ou affecter la stabilité du vecteur.

Dans ces conditions, le manque de promoteurs homologues forts chez Kluyveromyces constitue un facteur limitant dans l'exploitation industrielle de ce système d'expression.

La Demanderesse a maintenant identifié, cloné et séquencé une région du génome de Kluyveromyces lactis présentant une activité de promoteur transcriptionnel (voir figure 1). Plus précisément, cette région correspond au promoteur du gène ADH4 de K.lactis (KlADH4) codant pour l'alcool déshydrogénase IV. Cette région, ou des dérivés ou fragments de celle-ci, peut être utilisée de manière très performante pour la production de protéines recombinantes chez les levures du genre Kluyveromyces. Il est entendu que cette séquence peut également être utilisée dans d'autres organismes hôtes.

Par ailleurs, l'analyse de la région du génome de Kluyveromyces obtenue a permis de mettre en évidence une activité promotrice bidirectionnelle. Cette observation indique que le brin complémentaire de la région présentée sur la figure 1 possède également une activité promotrice agissant dans l'autre orientation.

De plus, un autre avantage de l'activité promotrice obtenue réside dans son caractère régulable. De ce fait, selon les conditions d'utilisation (milieu, souche), il est possible de contrôler l'activité du promoteur, et donc de déclencher ou de réprimer l'expression d'un gène recombiné.

Un autre avantage de la région promotrice obtenue réside dans l'absence de répression par le glucose. Ce résultat est surprenant puisqu'il s'agit du premier exemple de promoteur dérivé d'un gène ADH induit par l'éthanol et non réprimé par le glucose. En effet, dans le cas de S.cerevisiae. le gène ADH2 est exprimé sur différentes sources de carbone non fermentescibles (glycérol, éthanol, lactate, pyruvate), mais, contrairement à KlADH4, l'expression de ce gène est très fortement réprimée lorsque du glucose est ajouté au milieu (Denis et al., J. Mol. Biol. 148 (1981) 355). De même, l'expression du gène alcA qui code pour une alcool deshydrogénase I chez Aspergillus nidulans est induite par l'éthanol, mais sensible à la répression par le glucose (Falenbok, J.Biotechnol. 17 (1991)11).

Un objet de la présente invention réside donc dans une séquence d'ADN comprenant tout ou partie de la séquence présentée à la figure 1 ou de son brin complémentaire, ou d'un dérivé de celles-ci, et possédant une activité de promoteur transcriptionnel.

Au sens de la présente invention, on entend par dérivé, toute séquence obtenue à partir de la séquence donnée dans la figure 1 par modifications structurales (mutations, délétions, substitutions, additions, fragmentations ..) conservant une activité de promoteur. En particulier, les mutations peuvent porter sur un ou plusieurs nucléotides, et les additions et/ou substitutions peuvent porter sur des éléments de régulation, ou des régions activatrices telles que les "UAS".

Lorsqu'un dérivé est réalisé, son activité de promoteur transcriptionnel pot être mise en évidence de plusieurs façons, et en particulier en plaçant sous le contrôle de la séquence étudiée, un gène de résistance ou un marqueur de complémentation. Toute autre technique connue de l'homme de l'art pot bien évidemment être utilisée à cet effet.

Comme exemples de dérivés selon l'invention, on peut citer plus particulièrement un promoteur comprenant le fragment SacI-BamHI de 500 pb environ dont la séquence est présentée sur la figure 2 ou un fragment SalI-HindIII de 700 pb environ dont la séquence est présentée sur la figure 3, ou le fragment BglII-SacI de 700 pb environ correspondant au fragment compris entre les nucléotides 1 et 723 du brin complémentaire de la séquence présentée sur la figure 1.

La construction de ces promoteurs est décrite en détail dans les exemples.

La séquence présentée sur la figure 1 a été obtenue à partir d'un fragment BamHI de 8 kb environ, obtenu par criblage d'une banque d'ADN génomique total de Kluyveromyces lactis au moyen d'une sonde hétérologue provenant du gène de structure ADH2 de S.cerevisiae. La Demanderesse a en effet montré qu'il est possible de cloner une région promotrice chez Kluyveromyces, par hybridation à partir de sondes hétérologues correspondant à un gène de S.cerevisiae. Les détails du clonage de la séquence sont donnés dans les exemples. Les dérivés selon l'invention peuvent ensuite être préparés à partir de ces séquences, comme indiqué dans les exemples.

Un autre objet de l'invention concerne un ADN récombinant comprenant une séquence d'ADN telle que définie ci-dessus.

Cet ADN recombinant peut contenir par exemple la séquence promotrice présentée à la figure 1 ou un dérivé de celle-ci, dans laquelle est inséré un site de restriction, facilitant l'utilisation de cette séquence comme promoteur "portable".

Préférentiellement, cet ADN recombinant contient en outre un ou plusieurs gènes de structure. En particulier, il peut s'agir de gènes codant pour des protéines d'intérêt pharmaceutique ou agroalimentaire. A titre d'exemple, on peut citer les enzymes (tels que notamment la superoxide dismutase, la catalase, les amylases, les lipases, les amidases, la chymosine etc.), les dérivés sanguins. (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies [G-CSF, GM-CSF, M-CSF...], le TNF, le TRF etc.), les facteurs de croissance (tels que l'hormone de croissance, l'érythropoïétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus [CD4, etc.]).

Encore plus préférentiellement, l'ADN recombinant contient également des signaux permettant la sécrétion du produit d'expression du ou desdits gènes de structure. Ces signaux peuvent correspondre aux signaux naturels de sécrétion de la protéine considérée, mais ils peuvent également être d'une origine différente. En particulier des signaux de sécrétion dérivés de gènes de levure peuvent être utilisés, tels que ceux des gènes de la toxine killer (Stark et Boyd, EMBO J. 5 (1986) 1995) ou de la phéromone alpha (Kurjan et Herskowitz, Cell 30 (1982) 933 ; Brake et al., Yeast 4 (1988) S436).

Dans un mode de réalisation particulier de l'invention, l'ADN recombinant fait partie d'un plasmide d'expression, qui peut être à réplication autonome ou intégratif.

En particulier, des vecteurs à réplication autonome peuvent être obtenus en utilisant des séquences à réplication autonomes chez l'hôte choisi. Notamment, chez la levure, il peut s'agir d'origines de réplication dérivées de plasmides (pKD1, 2μ, etc), ou bien de séquences chromosomiques (ARS).

Les vecteurs intégratifs peuvent être obtenus notamment en utilisant des séquences homologues à certaines régions du génome de l'hôte, permettant, par recombinaison homologue, l'intégration du vecteur.

Un autre objet de l'invention concerne les cellules recombinées contenant une séquence d'ADN telle que définie ci-avant.

Avantageusement, les cellules sont choisies parmi les levures, et encore plus préférentiellement, parmi les levures du genre Kluyveromyces. Il est entendu cependant que l'invention couvre toutes les cellules recombinées dans lesquelles les régions promotrices de l'invention sont actives.

Ces cellules peuvent être obtenues par toute méthode permettant d'introduire un ADN étranger dans une cellule. Il peut s'agir notamment de transformation, électroporation, ou toute autre technique connue de l'homme de l'art.

Un autre objet de l'invention connue l'utilisation d'une séquence telle que précédemment définie pour l'expression de gènes recombinés.

Comme l'illustrent les exemples, les séquences d'ADN selon l'invention permettent en effet une production à des niveaux élevés de protéines recombinantes.

Par ailleurs, l'activité promotrice bidirectionnelle des séquences de l'invention permet une utilisation particulièrement avantageuse. Notamment, il est possible d'utiliser ces séquences pour l'expression simultanée de plusieurs gènes recombinés dans les deux orientations opposées.

Avantageusement, l'invention concerne l'utilisation d'une séquence telle que précédemment définie pour l'expression simultanée de deux gènes recombinés insérés de part et d'autre du promoteur, dans les deux orientations opposées.

Avantageusement, les séquences de l'invention peuvent être utilisées pour l'expression de gènes codant pour des protéines d'intérêt pharmaceutique ou agroalimentaire. A titre d'exemple, on peut citer les protéines énumérées précédemment.

La présente invention permet également la réalisation d'un procédé de production de protéines recombinantes, selon lequel on cultive une cellule recombinée telle que définie ci-avant et on récupère la protéine produite. A titre d'exemple de protéine, on peut citer les protéines énumérées précédemment.

Préférentiellement, le procédé de l'invention est applicable à la production de sérum-albumine humaine, ou un de ses variants moléculaires. On entend par variant moléculaire de l'albumine, les variants naturels résultant du polymorphisme de l'albumine, les formes tronquées, ou toute protéine hybride à base d'albumine.

Par ailleurs, un aspect particulièrement avantageux de l'invention réside dans la possibilité de réguler l'activité des promoteurs. La demanderesse a en effet montré que l'activité promotrice était spécifiquement induite par l'éthanol. A cet effet, l'éthanol inducteur peut soit être ajouté directement au milieu de culture, soit être produit intracellulairement par la souche hôte, selon ses capacités de fermentation, à partir de la source de carbone introduite dans le milieu. La régulation peut donc être obtenue dans plusieurs conditions:

- soit en cultivant la cellule récombinée dans un milieu contenant une source carbonée différente de l'éthanol et non transformable en éthanol par la cellule. Dans ce cas, l'activité du promoteur est induite par addition d'éthanol dans le milieu. Par exemple, dans le cas de K.lactis 2359/152 et K.lactis MW98-8C, le promoteur KlADH4 est inactif lorsque les cellules sont cultivées sur un milieu contenant du glycérol, mais il est induit après addition d'éthanol.
- soit en cultivant, dans un milieu contenant une source carbonée fermentescible (par exemple le glucose), une cellule recombinée présentant une déficience au niveau de son métabolisme fermentatif, et de ce fait, incapable de produire de l'éthanol à partir de cette source carbonée. Dans ce cas, l'activité du promoteur est induite soit par addition d'éthanol dans le milieu, soit par addition d'un autre sucre fermentescible intervenant dans une étape en aval de celle déficiente, et capable d'être métabolisé en éthanol en dépit de ladite déficience. A titre d'exemple, on peut utiliser une souche présentant une mutation rag2, et de ce fait ne possédant pas l'activité phosphoglucose isomérase. Dans ce cas, le promoteur KlADH4 est inactif sur milieu glucose, et l'activité du promoteur peut être induite par addition d'éthanol ou de fluctose.

D'autres avantages de la présente invention apparaîtront à la lecture des exemples qui suivent, qui doivent être considérés comme illustratifs et non limitatifs.

## LEGENDE DES FIGURES

Figure 1: Séquence nucléotidique du fragment de 1,2 kb correspondant au promoteur KlADH4 de K.lactis.
Figure 2: Séquence nucléotidique du fragment SacI-BamHI de 0,5 kb environ, correspondant au promoteur KlADH4 tronqué dans le vecteur pP4-15Kan.
Figure 3: Séquence nucléotidique du fragment SalI-HindIII de 0,7 kb environ, correspondant au promoteur KlADH4 tronqué dans les vecteurs pYG129 et pYG130.
Figure 4: Représentation schématique du plasmide p6-4-98 et construction des plasmides p6-2200-2 et pP4-33.
Figure 5: Représentation schématique du plasmide pSK-Kan401 et construction des plasmides pP4Kan et pP4R-Kan.
Figure 6: Construction et représentation des plasmides pP4-GUS et pSK-GUS.
Figure 7: Représentation schématique du plasmide pKan707 et construction des dérivés du phage M13, pYG64 et pYG65.
Figure 8: Construction et représentation des plasmides pYG69 et pYG70.
Figure 9: Construction et représentation du plasmide pYG72.
Figure 10: Représentation schématique du plasmide pYG404 et construction du plasmide pYG404ΔH.
Figure 11: Construction et représentation du plasmide pYG128.
Figure 12: Construction et représentation des plasmides pYG131 et pYG132.
Figure 13: Construction et représentation des plasmides pP4-15Kan et pP4-60Kan.

Figure 14: Construction et représentation des plasmides pYG129 et pYG130.

Figure 15 : Gel de polyacrylamide natif (5 %) coloré selon le protocole décrit dans l'exemple 7.1.2. et démontrant l'expression de l'activité β-glucuronidase dans la souche MW98-8C transformée avec le plasmide pP4-GUS (lignes 1-6 correspondant à 6 clones indépendants). Les résultats obtenus à partir de la souche MW98-8C transformée avec le plasmide pSK-GUS (témoin) sont donnés aux lignes C.

Figure 16: Gel de polyacrylamide-SDS à 8,5 % après coloration au bleu de Coomassie démontrant la sécrétion de la sérum albumine humaine à partir de la souche MW98-8C transformée avec le vecteur pYG132. Pistes a-c, albumine extrait du plasma humain (Sigma) utilisée comme standard et déposée à des concentrations croissantes (0,5, 1,0 et 1,5 μg par piste). Les autres pistes correspondent à 25 μl de surnageant de culture obtenus à partir des milieux décrits dans l'exemple 7.2.1. Glu, glucose ; Glu/EtOH, glucose/éthanol ; Gly, glycérol ; Gly/EtOH, glycérol/éthanol.

Figure 17: Gel de polyacrylamide-SDS à 8,5 % après coloration au bleu de Coomassie démontrant la sécrétion de la sérum albumine humaine à partir de la souche CBS 293.91 transformée avec le vecteur pYG132. Pistes a-c, albumine extrait du plasma humain (Sigma) utilisée comme standard et déposée à des concentrations croissantes (0,5, 1,0 et 1,5 μg par piste). Les autres pistes correspondent à 25 μl de surnageant de culture obtenus à partir des milieux décrits dans l'exemple 7.2.1. Glu, glucose ; Glu/EtOH, glucose/éthanol ; Gly, glycerol ; Gly/EtOH, glycérol/éthanol.

Figure 18: Gel de polyacrylamide-SDS à 8,5 % après coloration au bleu de Coomassie démontrant la sécrétion de la sérum albumine humaine à partir de la souche CBS 293.91 transformée avec le vecteur pYG130. Pistes a-b, albumine extrait du plasma humain (Sigma) utilisée comme standard et déposée à des concentrations croissantes (0,5 et 1,0 μg par piste). Les autres pistes correspondent à 25 μl de surnageant de culture obtenues à partir des milieux décrits dans l'exemple 7.2.1. Glu, glucose; Glu/EtOH, glucose/éthanol.

Tableau 1: Résumé des différentes constructions effectuées à partir du promoteur KlADH4 entier (fragment de 1,2 kb, soit sous forme d'un fragment BglII-BamHI [pP4-Kan, pP4R-Kan, et pP4-GUS], soit sous forme d'un fragment SalI-HindIII [pYG131 et pYG132]).

Tableau 2: Résumé des différentes constructions effectuées à partir du promoteur KlADH4 tronqué (fragment SacI-BamHI de 0,5 kb dont la séquence nucléotidique est donnée à la figure 2 [pP4- 15Kan] ; fragment BglII-SacI de 0,7 kb correspondant aux positions 1-723 de la séquence nucléotidique donnée à la figure 1 [pP4-60Kan] ; fragment SalI-HindIII de 0,67 kb dont la séquence nucléotidique est donnée à la figure 3 [pYG129 et pYG130]).

Tableau 3: Etude de la régulation de l'expression du gène KlADH4. La présence ou absence de l'activité enzymatique ADH IV mise en évidence selon le protocol décrit dans l'exemple 5 est indiquée par les symboles + ou -.

## TECHNIQUES GENERALES DE CLONAGE

Les méthodes claniques de biologie moléculaire telles que la centrifugation d'ADN plasmidique en gradient de chlorure de césium - bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning : a Laboratory Manual", Cold Spring Harbor Laboratory, Cold Spring Harbor, N.Y., 1986 ; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain réaction") dans les conditions décrites par Mullis et Faloona (Meth. Enzym., 155 (1987) 335-350) et Saiki et al (Science 230 (1985) 1350-1354), en utilisant un "DNA thermal cycler" (Perkin Elmer Cetus) en suivant les recommandations du fabricant.

## EXEMPLES

Plasmides et souches utilisés dans les exemples.

Les plasmides suivants ont servi aux différentes étapes de clonage, ou lors de la construction de vecteurs d'expression.

- Vecteur de remplacement Lambda-L47 : Loenen et Brammar, Gene 10 (1980) 249-259;

- Plasmide pBR322 (Pharmacia, Uppsala, Suède) ;
- Plasmide pTZ19 (Pharmacia, Uppsala, Suède);
- Plasmide pSK-Kan401: Chen et Fukuhara, Gene 69 (1988) 181-192;
- Plasmide pBI221 (Clontech Laboratories, Palo Alto, CA, USA) ;
- Plasmide pBC SK +/- (Stratagène, La Jolla, CA, USA) ;
- Plasmide pYG404 (EP 361 991);
- Plasmide pKan707 (EP 361 991);
- Bactériophage M13mp7 : Messing et al., Proc. Natl. Acad. Sci. USA 74 (1977) 3652.

Les souches suivantes ont été utilisées pour le clonage, la construction et l'utilisation des promoteurs de l'invention.

- K.lactis CBS2359/152 (n° CBS 289.91)
- K.lactis MW98-8C (n° CBS 579.88)
- K.lactis CBS 293.91

**1/Isolement du promoteur KIADH4 de K.lactis.**

La séquence présentée sur la figure 1 a été obtenue par criblage d'une banque d'ADN génomique total de Kluyveromyces lactis CBS2359/152 au moyen d'une sonde hétérologue provenant du gène ADH2 de S.cerevisiae. Plus précisément, la banque a été obtenue par clonage au site BamHI du vecteur de remplacement Lambda-L47 du produit d'une digestion partielle par l'enzyme Sau3A de l'ADN de K.lactis CBS2359/152. La sonde utilisée pour l'hybridation est un fragment EcoRV-BamHI de 980 bp comprenant la région codante du gène de structure ADH2 de S.cerevisiae, à l'exception des 70 premières pb (sonde A). Ce fragment est obtenu par digestion enzymatique à partir d'un plasmide nommé pBR322.ADR2.BSa (Williamson et al., Cell 23 (1981) 605-614 ; Russell et al., J. Biol.Chem.,258 (1983) 2674-2682).

Un fragment de 8 kb environ a ainsi été isolé et sous-cloné au site BamHI du plasmide pBR322 pour générer le plasmide p6-4-98 (figure 4). L'insert BamHI porté par ce plasmide a ensuite été cartographié au moyen d'enzymes de restriction, et la région promotrice du gène KlADH4 a été localisée sur ce fragment par hybridations différentielles en utilisant la sonde A ainsi qu'une deuxième sonde correspondant au fragment BamHI-EcoRV de 1100 pb environ du plasmide pBR322.ADR2.BSa (sonde B).

Dans une seconde étape, le plasmide p6-4-98 a été digéré par l'enzyme HindIII et un fragment de 2,2 kb a été isolé. Ce fragment a ensuite été purifié par des techniques standard et sous-cloné au site HindIII du plasmide pTZ19 pour générer le plasmide p6-2200-2 (figure 4). L'analyse du fragment sous-cloné révèle qu'il contient les 14 premiers codons du gène KlADH4 et la région située en amont de celui-ci, comprenant les éléments de régulation de l'expression.

La partie comprise entre le site BglII et le codon d'initiation de la traduction ATG (fragment de 1,2 kb environ) a été séquencée en utilisant la méthode de terminaison de chaine (Sanger et al., Proc.Nat.Acad.Sci. 74 (1977) 5463). La séquence de ce fragment est présentée sur la figure 1.

**2/Construction d'un promoteur KIADH4 portable (BglII-BamHI)**

Un promoteur portable a été préparé par insertion sur le fragment HindIII de 2,2 kb présent sur le plasmide p6-2200-2 d'un site de restriction BamHI en position - 16 par rapport au codon ATG du gène KlADH4.

L'insertion de ce site permet de générer un fragment BglII-BamHI de 1,2 kb comprenant exclusivement la région promotrice KlADH4. Elle permet également d'introduire en aval du promoteur ainsi obtenu tout gène que l'on désire exprimer.

Le site BamHI a été introduit en position -16 par rapport au site d'initiation de la traduction (ATG) du gène KlADH4 par mutagénèse dirigée en utilisant la technique de double amorce (Sambrook, Fritsch, Maniatis, Molecular Cloning Laboratory Manual, Cold Spring Harbor Lab Press, 1989). La séquence des oligodésoxynucléotides synthétiques utilisés pour cette mutagénèse est donnée ci-dessous. Le site BamHI généré est souligné, l'ATG est indiqué en italiques et les astérisques désignent les bases modifiées par rapport à la séquence initiale. SI = Séquence initiale; SM = Séquence modifiée.

5'-CTCCCCCACCAACAACACAACATACAACACACGCA*ATG*TTCAGATT-3'(SI)

** *

5'-CTCCCCCACCAACAACACAGGATCCAACACACGCA*ATG*TTCAGATT-3'(SM)
3'-GAGGGGGTGGTTGTTGTGTCCTAGGTTGTGTGCGTTACAAGTCTAA-5'(SM)

Le plasmide ainsi obtenu est appelé pP4-33 (figuré 4).

## 3/Construction de vecteurs de clonage ou d'expression contenant le promoteur entier.

Le tableau 1 rassemble les constructions décrites dans cet exemple.

### 3.1.Construction d'un vecteur d'expression du gène aph :

L'ADN du plasmide pP4-33 (Cf exemple 2) a été digéré par BglII et BamHI pour générer le fragment de 1,2 kb contenant le promoteur KlADH4. Ce fragment a ensuite été introduit au site BamHI du plasmide pSK-Kan401, en amont du gène reporteur aph codant pour l'aminoglycoside-3'-phosphotransférase (I) (Oka et al., J. Mol. Biol. 147 (1981) 217) dépourvu de son propre promoteur, qui, lorsqu'il est exprimé, confère à la levure la résistance à la généticine (G418) (Jimenez et Davies, ref). Après amplification chez E.coli, 2 plasmides recombinants ont été obtenus, différant au niveau de l'orientation de l'insert. Dans le plasmide pP4-Kan le fragment de 1,2 kb est dans la même orientation par rapport au gène aph que celle observée dans le contexte ADH4, alors qu'il se trouve dans l'orientation opposée sur le plasmide pP4R-Kan (voir figure 5).

### 3.2. Construction d'un vecteur d'expression des gènes GUS et aph :

Le plasmide pP4R-Kan a été utilisé pour construire un vecteur d'expression dans lequel 2 gènes hétérologues privés de leurs promoteurs respectifs sont placés de part et d'autre du promoteur KlADH4 de 1,2 kb. Les gènes hétérologues utilisés sont plus spécifiquement:

- le gène aph dont l'expression confère la résistance au G418, et
- le gène de la β-glucuronidase de E.coli (gène GUS), dont l'expression peut être mise en évidence par réaction enzymatique.

Pour réaliser cette construction, le plasmide pP4R-Kan a été digéré par EcoRI et BamHI. Par ailleurs, à partir du plasmide pBI221, le gène de la β-glucuronidase de E.coli a été isolé sous forme d'un fragment BamHI-EcoRI de 2,1 kb. Ce dernier a ensuite été introduit par ligation dans le plasmide pP4R-Kan linéarisé comme indiqué pour générer le plasmide pP4-GUS (voir figure 6). Parallèlement, un plasmide contrôle a été préparé par insertion du fragment BamHI-EcoRI de 2,1 kb issu du plasmide pBI221 portant le gène GUS dans le plasmide pSK-Kan401 préalablement digéré par BamHI et EcoRI. Le plasmide ainsi obtenu est appelé pSK-GUS (figure 6). Cette construction diffère du plasmide pP4-GUS uniquement par l'absence du promoteur KlADH4 de 1,2 kb.

### 3.3. Construction d'un vecteur d'expression du gène codant pour la sérum albumine humaine (HSA) :

Pour construire différents vecteurs d'expression de la sérum-albumine humaine, un dérivé du plasmide pYG404 (Cf EP 361 991) a été préparé, contenant:

- un réplicon de levure (séquence quasiment entière du plasmide naturel pKD1),
- le gène codant pour la prépro-albumine humaine (HSA) sous contrôle du promoteur du gène LAC4 de K.lactis et suivi du terminateur du gène PGK de S.cerevisiae; le gène de structure codant pour la HSA est précédé par une séquence de 25 nucléotides correspondant à la région directement en amont du gène PGK de S.cerevisiae,
- le gène aph conférant la résistance à la généticine (G418) à la levure, et
- un réplicon et un marqueur de sélection (gène bla conférant la résistance à l'ampicilline) pour E.coli.

Ce plasmide, nommé pYG404ΔH, diffère de pYG404 uniquement par la destruction du site HindIII, localisé dans le gène aph, par mutagénèse dirigée. Cette modification a ensuite permis de substituer le promoteur LAC4 présent dans le plasmide pYG404ΔH sous forme d'un fragment SalI-HindIII par différents variants du promoteur KlADH4 égalements construits comme promoteurs portables sous forme de fragments SalI-HindIII.

*3.3.1 Construction du plasmide pYG40ΔH (figures 7-10).*

Pour effectuer la délétion du site HindIII dans le vecteur de clonage pYG404, différentes étapes de sous-clonage ont été effectuées, donnant lieu à une construction intermédiaire : pYG72 (figure 9). Ce vecteur correspond au plasmide pKan707 (EP 361 991) dans lequel le fragment SacI contenant le gène URA3 a été éliminé ainsi que le site unique HindIII présent dans le gène aph. Pour effectuer la mutagénèse dirigée sur ce site, le fragment PstI de 1,3 kb portant le gène aph a été sous-cloné à partir du plasmide pKan707 dans le bactériophage M13mp7 pour donner le vecteur pYG64 (figure 7). Le site HindIII a été détruit par mutagénèse dirigée (Cf techniques générales de clonage) en utilisant l'oligodésoxynucléotide suivant : 5'-GAA ATG CAT AAG CTC TTG CCA TTC TCA CCG-3', permettant le remplacement du triplet CTT codant pour la leucine 185 par le triplet CTC. Ce changement ne modifie pas la séquence protéique résulante. Le plasmide obtenu a été appelé pYG65 (figure 7). Pour construire le plasmide pYG72, la partie contenant le réplicon bactérien du vecteur pKan707 a été isolée par digestion avec l'enzyme EcoRI et recircularisation avec la T4 DNA ligase, générant le plasmide intermédiaire pYG69. Le fragment PstI présent dans celui-ci contenant le gène aph a ensuite été remplacé par le fragment équivalent muté provenant du plasmide pYG65. Cette construction a été appelée pYG70 (figure 8). La séquence de pKD1 de 4,7 kb comprise entre les sites EcoRI et SacI a ensuite été introduite dans ce vecteur pour obtenir pYG72 (figure 9).

Le vecteur pYG404ΔH a été obtenu en insérant la cassette d'expression provenant du plasmide pYG404 (EP 361 991) sous forme d'un fragment SalI-SacI aux sites correspondants de pYG72 (figure 10).

*3.3.2. Construction d'un promoteur KlADH4portable [SalI-HindIII] (figure 11):*

Le promoteur KlADH4 porté sur le fragment BglII-BamHI provenant du plasmide pP4-33 (exemple 2) a été modifié de la manière suivante pour l'adapter à l'utilisation dans des vecteurs d'expression dérivés du plasmide pYG404ΔH :

Après digestion du plasmide pP4-33 par les enzymes BglI et BamHI suivi d'un traitement à la nucléase 'Mung Bean' pour rendre les extrémité franches, le fragment de 1,2 kb portant le promoteur KlADH4 a été isolé à partir d'un gel d'agarose et sous-cloné dans le vecteur pBC SK+ (Stratagene, La Jolla, CA, USA) préalablement linéarisé avec l'enzyme ClaI et traité à la nucléase 'Mung Bean' ainsi qu'à la phosphatase akaline de veau (CIP). Le plasmide obtenu de cette manière (pYG128, figure 11) permet l'isolement du promoteur KlADH4 sous forme d'un fragment SalI-HindIII de 1,2 kb.

*3.3.3. Construction des vecteurs pYG131 et pYG132 (figure 12):*

La digestion du vecteur d'expression pYG404ΔH (exemple 3.3.1.) par les enzymes SalI et HindIII permet le remplacement du promoteur LAC4 par le promoteur KlADH4 décrit ci-dessus.

Pour effectuer ce clonage, le fragment SalI-HindIII de 8,7 kb contenant la partie pKD1 et les marqueurs de sélection ainsi que le fragment HindIII-HindIII de 1,9 kb portant le gène codant pour la prépro-HSA ont été isolés à partir du vecteur pYG404ΔH et religués en présence du fragment SalI-HindIII de 1,2 kb provenant du plasmide pYG128 et portant le promoteur KlADH4. Deux plasmides ont été obtenus de cette manière:

- pYG131 (figure 12), correspondant à un vecteur de clonage permettant l'insertion au site HindIII unique de tout gène que l'on désire exprimer sous contrôle du promoteur KlADH4, et
- pYG132 (figure 12), qui est identique au plasmide pYG131 sauf qu'il contient le gène de la prépro-HSA introduit au site HindIII.

**4/Construction de vecteurs de clonage ou d'expression contenant des dérivés tronqués du promoteur KlADH4.**

Le tableau 2 rassemble les constructions décrites dans cet exemple.

**4.1.Construction de vecteurs d'expression du gène aph.**

Les plasmides pP4-Kan et pP4R-Kan (exemple 3.1.) ont été utilisés pour préparer des constructions contenant des formes réduites du promoteur. Pour cela, les plasmides pP4-Kan et pP4R-Kan ont été digérés par l'enzyme SacI puis religaturés, selon le schéma décrit sur la figure 13.

Cette manipulation a permis:

- d'exciser du plasmide pP4-Kan le fragment SacI de 0,7 kb environ situé du coté opposé au gène aph. Dans le plasmide obtenu (pP4-15Kan) le gène aph est donc sous le contrôle d'un promoteur réduit correspondant au fragment SacI-BamHI de 0,5 kb dont la séquence est donnée sur la figure 2, dans la même orientation que celle observée dans le contexte ADH4.
- d'exciser du plasmide pP4R-Kan le fragment SacI de 0,5 kb environ situé du coté opposé au gène aph. Dans le plasmide obtenu (pP4-60Kan) le gène aph est donc sous le contrôle d'un promoteur réduit correspondant au brin complémentaire du fragment BglII-SacI de 0,7 kb (nucléotides 1 à 723) qui fait partie de la séquence donnée sur la figure 1.

### 4.2. Construction d'un vecteur d'expression du gène de la prépro-HSA :

*4.2.1. Construction d'un promoteur KlADH4 tronqué portable [SalI-HindIII] (figure 14) :*

Un dérivé tronqué du fragment BglII-BamHI de 1,2 kb provenant du plasmide pP4-33 et portant le promoteur KlADH4 entier a été obtenu par amplification enzymatique (PCR) d'une partie de ce promoteur, située entre le site BsmAI en position 541 sur la séquence donnée sur la figure 1 et le site BamHI en position -16 par rapport à l'ATG du gène ADH4. Les oligodesoxynucléotides utilisés pour la réaction PCR étaient :

- 5'-GGGGTCGACGCGAGACAACACTATTGTGAG-3', introduisant un site SalI (séquence soulignée) juste en amont du site BsmAI précité, et
- 5'-GGGAAGCTTTGTGTTGTTG***ATG***GGGGAG -3', remplaçant le site BamHI présent dans la construction pP4-33 par un site HindIII (séquence soulignée).

*4.2.2. Construction des vecteurs pYG129 et pYG130*

Le fragment de 672 bp obtenu par PCR a été digéré par les enzymes SalI et HindIII, purifié à partir d'un gel d'agarose et ligué avec le fragment SalI-HindIII de 8,7 kb contenant la partie pKD1 et les marqueurs de sélection provenant du vecteur pYG404ΔH. Cette ligation a été effectuée en présence du fragment HindIII-HindIII de 1,9 kb portant le gène codant pour la prépro-HSA isolé à partir du même vecteur. Deux plasmides ont été obtenus de cette manière:

- pYG 129 (figure 14), correspondant à un vecteur de clonage permettant l'insertion au site HindIII unique de tout gène que l'on désire exprimer sous contrôle du promoteur tronqué KlADH4, et
- pYG130 (figure 14), qui est identique au plasmide pYG129 sauf qu'il contient le gène de la prépro-HSA introduit au site HindIII.

La séquence nucléotidique du fragment SalI-HindIII de 0,7 kb environ correspondant au promoteur KlADH4 tronqué utilisé dans ces deux constructions est donnée sur la figure 3.

### 5/Etude de la régulation de l'expression du gène KlADH4.

Cette étude a été réalisée avec les souches K.lactis MW98-8C et K.lactis 2359/152.

La souche MW98-8C présente un phénotype Rag2$^-$ dû à une mutation (rag2) au niveau du gène codant pour la phosphoglucose isomérase (PGI), qui la rend incapable de produire de l'éthanol sur un milieu glucose. La souche 2359/152 est Rag2$^+$.

L'activité du promoteur ADH4 a été déterminée dans différentes conditions de culture, par mise en évidence de l'activité ADH IV en utilisant la technique décrite par Lutstorf et Megnet (Archiv. Biochem. Biophys. 126 (1968) 933). Les résultats obtenus sont rassemblés dans le tableau 3.

Ces résultats indiquent que l'activité du promoteur KlADH4 est spécifiquement induite par l'éthanol et non déreprimée en l'absence du glucose. En effet, nous avons trouvé, d'une manière surprenante, que, contrairement aux promoteurs d'autres alcool deshydrogénases tel que ADH2 de S.cerevisiae ou alcA d'A. nidulans, le promoteur KlADH4 n'est pas réprimé par le glucose (tableau 3). L'éthanol inducteur peut soit être ajouté au milieu de culture soit être produit intracellulairement par fermentation d'une source carbonée tel que le glucose ou le fructose (tableau 3).

Nous avons également montré qu'une souche mutée dans un gène impliqué dans la glycolyse et pour cette raison incapable de prodiure de l'éthanol à partir du glucose peut être avantageusement utilisée comme hôte pour les vecteurs d'expression de l'invention. En effet, le promoteur KlADH4 est inactif dans un milieu contenant du glucose comme seule source de carbone dans des souches tels que MW98-8C (rag2) dont le gène codant pour la phosphoglucose isomérase est défectif. Dans cette souche, l'activation du promoteur KlADH4 s'effectue par l'addition d'éthanol au milieu de culture.

Par ailleurs, nous avons trouvé, d'une manière surprenante, que dans les souches CBS2359/152 et MW98-8C le promoteur KlADH4 est inactif dans un milieu contenant du glycérol comme seule source de carbone. Ce résultat diffère de ce qui est connu du promoteur du gène de l'alcool deshydrogénase II de S.cerevisiae (ADH2) qui est actif sur différentes sources de carbone non fermentescibles incluant le glycérol.

Ces résultats montrent donc que certaines souches tel que CBS2359/152, même avec un phénotype Rag$^+$, peuvent permettre l'obtention d'une expression régulée du promoteur KlADH4 : les cellules cultivées en présence de glycérol comme seule source de carbone ne produisent pas la protéine dont le gène est placé sous contrôle du promoteur KlADH4 (promoteur non-induit) tandis qu'elles peuvent être induite pour la production de cette protéine en ajoutant de l'éthanol au milieu de culture.

**6/Transformation de Kluyveromyces.**

Différentes techniques permettant l'introduction d'ADN dans la levure peuvent être utilisées.

Avantageusement, les différentes souches de Kluyveromyces utilisées ont été transformées en traitant les cellules entières en présence d'acétate de lithium et de polyéthylène glycol, selon la technique décrite par Ito et al. (J. Bacteriol.153 (1983) 163-168). La technique de transformation décrite par Durrens et al. (Curr.Genet. 18 (1990) 7) utilisant l'éthylène glycol et le diméthylsulfoxyde a également été utilisée. Il est aussi possible de transformer les levures par électroporation, par exemple selon la méthode décrite par Karube et al. (FEBS Letters 182 (1985) 90).

Un protocole alternatif a encore été décrit en détail dans la demande EP 361 991.

**7/Utilisation de vecteurs d'expression pour la production de protéines recombinantes.**

**7.1. Production de protéines bactériennes.**

*7.1.1. Production de l'aminoglycoside 3'-phosphotransférase (I)*

Les plasmides d'expression pP4-Kan et pP4R-Kan (exemple 3.1) ainsi que le plasmide de clonage pSK-Kan401 (témoin) ont été utilisés pour transformer la levure K. lactis MW98-8C. Les cellules transformées par pP4-Kan et pP4R-Kan possèdent la résistance au G418 dans un milieu YPD (extrait de levure 10 g/l ; peptone 20 g/l ; glucose 20 g/l) contenant 2 % d'éthanol et des doses de généticine allant de 200 à 400 µg/ml, alors que les cellules transformées par le plasmide pSK-Kan401 sont sensibles à la généticine.

Ce résultat indique:

- que le gène aph est exprimé dans les levures transformées par les plasmides pP4-Kan et pP4R-Kan, ce qui démontre que le fragment de 1,2 kb porte bien une activité promotrice fontionnelle, et,
- que ce fragment porte donc bien une activité promotrice bidirectionnelle, puisque le gène aph est exprimé dans les deux constructions, c'est-à-dire quelle que soit l'orientation du fragment BamHI-BglII de 1,2 kb contenant le promoteur KlADH4.

Les plasmides pP4-15Kan et pP4-60Kan confèrent, après transformation, la résistance à la généticine à la souche MW98-8C cultivée dans un milieu YPD contenant 2 % d'éthanol et des doses de généticine supérieure à 200 µg/ml.

Ce résultat indique clairement que des dérivés actifs du promoteur KlADH4 porté par le fragment 1,2 kb peuvent être obtenus par fragmentation. Il confirme l'activité bidirectionnelle de ce promoteur.

*7.1.2. Production de la β-glucuronidase (figure 15)*

Les plasmides pP4-GUS et pSK-GUS (exemple 3.2) ont été utilisés pour transformer la souche K.lactis MW98-8C (ura3 lysA argA) en sélectionnant les cellules recombinées pour leur prototrophie vis-à-vis de l'uracile sur un milieu synthétique SD ('yeast nitrogen base w/o amino acids' 0,67 %, glucose 2 %) supplémenté avec de l'arginine (20 mg/l) et de la lysine (30 mg/l).

Les cellules recombinées ont été cultivées dans 10 ml de YPD jusqu'à la phase stationnaire. Les cellules ont ensuite été cassées au moyen de billes de verre dans des tubes Eppendorf, centrifugées, et les surnageants analysés par electrophorèse sur minigel (5 % d'acrylamide) en conditions non-dénaturantes. Les gels et tampons utilisés ont été décrits par Williamson et al. (Nature 283 (1980) 214-216).

Des échantillons correspondant à 20-40 µg de protéines ont été séparés par électrophorèse sur gel d'acrylamide (5 %). La migration a été réalisée à 4°C pendant 60 minutes sous un courant de 20 mA.

L'activité β-glucuronidase est mise en évidence en trempant les gels dans une solution colorante contenant 50 mM Na2HPO4, pH 7,0 et 50 µg/ml de bromo-5-chloro-4-indoyl-3 glucuronide (X-Glu) dans 5 mg/ml de diméthylformamide (Jefferson R.A., Plant. mol. Biol. Report 5 (1987) 387-405).

Les gels ont été maintenus à 37°C dans cette solution jusqu'à apparition des bandes.

Les résultats obtenus sont présentés sur la figure 15. Le gel représenté montre clairement une bande dans les lignes 1 à 6, correspondant aux cellules MW98-8C transformées par le plasmide pP4-GUS, et aucun signal dans les lignes C correspondant aux cellules MW98-8C transformées par le plasmide pSK-GUS, qui ne contient pas de région promotrice de l'invention.

Par ailleurs, les cellules transformées avec les plasmides pP4-GUS et pSK-GUS ont également été cultivées sur un milieu YPD supplémenté par de la généticine (200 mg/l). Les transformants contenant pP4-GUS ont acquis la résistance contre cette antibiotique tandis que la souche contrôle contenant pSK-GUS reste sensible.

Ces résultats confirment l'activité promotrice bidirectionnelle du fragment 1,2 kb. Ils montrent de plus que ce fragment peut être utilisé pour l'expression simultanée de deux gènes hétérologues insérés de part et d'autre du promoteur KlADH4, dans les 2 orientations opposées.

### 7.2. Production de protéines mammifères

*7.2.1. Expression du gène de la prépro-HSA sous contrôle du promoteur KlADH4 entier (figures 16-17) :*

Le plasmide d'expression pYG132 a été utilisé pour transformer les souches de levures K.lactis MW98-8C (Rag2$^-$, incapable de produire de l'éthanol à partir de glucose) et CBS 293.91 (Rag2$^+$, métabolisant le glucose pour produire de l'éthanol). Après sélection des cellules recombinantes sur milieu YPD supplémenté par de la généticine (200 mg/l), les transformants ont été précultivés pendant 20 heures environ dans des flacons Erlenmeyer dans un milieu M9EL10 (milieu M9 [Maniatis et al., précité] supplémenté de 10 g/l d'extrait de levure) en présence de glucose (20 g/l) à 28°C sous agitation. Cette préculture a ensuite été utilisée pour inoculer, à une dilution de $10^{-3}$, des Erlenmeyers de 300 ml contenant 50 ml de milieu M9EL10 en présence de différentes sources de carbone (soit glucose seul [20 g/l], soit glucose [20 g/l] plus éthanol [20 g/l], soit glycérol seul [20 g/l], soit glycérol [20 g/l] plus éthanol [20 g/l]).

Après culture des cellules recombinantes pendant 5 jours à 28°C sous agitation, des échantillons du surnageant de chaque culture, dépourvus de cellules, ont été prélevés et mélangés à un volume équivalent de tampon Laemmli 2X (Laemmli, Nature 227, [1970] 680). Après chauffage à 96° C pendant 10 minutes, les protéines contenues dans un équivalent de 25 µl de surnageant on été séparées (25 mA) sur gel de polyacrylamide SDS 8,5 %. La sécrétion d'albumine a ensuite été révélée par coloration du gel au bleu de Coomassie, et évaluée par densitométrie (densitomètre Shimazu CS930).

La figure 16 montre le résultat obtenu avec la souche MW98-8C/pYG132 pour laquelle on observe une sécrétion d'albumine à haut niveau quand les cellules ont été cultivées en présence d'éthanol ajouté au milieu de culture. Par contre, on ne détecte pas de HSA dans le surnageant quand les cellules ont été cultivées en présence de glucose ou de glycerol.

Contrairement aux résultats obtenus avec la souche MW98-8C/pYG132, les transformants CBS293.91/pYG132 sont capables de produire de la HSA dans toutes les conditions de cultures évoquées ci-dessus (figure 17). Néanmoins, la production d'albumine est 2 à 3 fois plus élevée dans une culture contenant de l'éthanol ajouté au milieu par rapport à une culture où l'éthanol est le produit du métabolisme cellulaire.

Ces résultats confirment la capacité des couples hôte/vecteurs décrits dans les exemples précédents à produire à des niveaux élevés, en condition d'induction, une protéine recombinante, qu'elle soit d'origine bactérienne ou mammifère. Par exemple, la production d'albumine en Erlenmeyers dans la souche CBS 293.91/pYG132 dans un milieu contenant du glycerol ou du glucose en présence d'éthanol (figure 17) a été estimée par densitométrie à 190 - 200 mg/l.

Ces résultats confirment également l'absence de répression du promoteur KlADH4 par le glucose puisque la production d'albumine n'est pas réduite en présence de celui-ci.

*7.2.2. Expression du gène de la prépro-HSA sous contrôle du promoteur KlADH4 tronqué (figure 18) :*

Le plasmide d'expression pYG130 a été utilisé pour transformer la souche K.lactis CBS 293.91 (Rag2$^+$). Après sélection des cellules recombinantes sur milieu YPD supplémenté par de la généticine (200 mg/l), les transformants ont été précultivés et cultivés comme décrit dans l'exemple précédent. La sécrétion d'albumine a été évaluée par électrophorèse d'échantillons de surnageants de culture comme décrit sous 7.2.1. Le résultat obtenu avec le dérivé tronqué du promoteur KlADH4 est montré à la figure 18: le couple hôte/vecteur CBS293.91/pYG130 est clairement capable de produire et sécréter de l'albumine recombinante. Comme dans l'exemple du plasmide pYG 132 (promoteur KlADH4 entier) on observe une production d'albumine dans le milieu contenant du glucose comme seule source de carbone, mais à un niveau moins élevé par rapport au promoteur entier. Par contre, la culture des cellules dans un milieu contenant du glucose plus de l'éthanol augmente la production de HSA d'un facteur 2 à 3 (figure 18).

Un échantillon de la souche K.lactis 2359/152 a été déposé le 4 juin 1991 auprès du Centraalbureau voor Schimmelkulturen (CBS) à Baarn aux Pays-Bas dans les conditions du Traité de Budapest, sous le numéro CBS 289.91. La souche K. lactis CBS 293.91 correspond à la souche CBS1065 redéposée le 11 juin 1991 selon les conditions du Traité de Budapest.

## TABLEAU 1

| VECTEUR | PLASMIDE NAVETTE | CASSETTE D'EXPRESSION |
|---|---|---|
| pP4-Kan | pSK-Kan401 | 1.2 > aph |
| pP4R-Kan | pSK-Kan401 | aph 1.2 > |
| pP4-GUS | pSK-Kan401 | aph 1.2 > GUS |
| pYG131 | pYG404D | 1.2 > NEANT |
| pYG132 | pYG404D | 1.2 > HSA |

## TABLEAU 2

| VECTEUR | PLASMIDE NAVETTE | CASSETTE D'EXPRESSION |
|---|---|---|
| P4-15Kan | pSK-Kan401 | 0,5 > aph |
| pP4-60Kan | pSK-Kan401 | aph 0,7 |
| pYG129 | pYG404DH | 0,67 > NEANT |
| pYG130 | pYG404DH | 0,67 > HSA |

## TABLEAU 3

| Souche\Milieu | Glucose | Ethanol | Glucose+ Ethanol | Fructose | Glycerol |
|---|---|---|---|---|---|
| 2359/152 (Rag2+) | + | + | + | + | - |
| MW98-8C (Rag2-) | - | + | + | + | - |

## Revendications

1. Séquence d'ADN comprenant tout ou partie de la séquence présentée à la figure 1 ou de son brin complémentaire, ou d'un dérivé de celles-ci, et possédant une activité de promoteur transcriptionnel.

2. Séquence d'ADN selon la revendication 1 comprenant tout ou partie du fragment SacI-BamHI de 0,5 kb environ présenté sur la figure 2 ou du fragment SalI-HindIII de 0,7 kb environ présenté sur la figure 3.

3. Séquence d'ADN selon la revendication 1 comprenant tout ou partie du fragment BglII-SacI de 0,7 kb environ correspondant au fragment compris entre les nucléotides 1 et 723 du brin complémentaire de la séquence donnée sur la figure 1.

4. ADN recombinant comprenant une séquence d'ADN selon l'une quelconque des revendications 1 à 3.

5. ADN recombinant selon la revendication 4 caractérisé en ce qu'il contient en outre un ou plusieurs gènes de structure.

6. ADN recombinant selon la revendication 5 caractérisé en ce qu'il contient également des signaux permettant la sécrétion du produit d'expression du ou desdits gènes de structure.

7. ADN recombinant selon les revendications 5 et 6 caractérisé en ce que le ou les gènes de structure codent pour des protéines d'intérêt pharmaceutique ou agroalimentaire.

8. ADN recombinant selon la revendication 7 caractérisé en ce que le ou les gènes de structure codent pour des protéines choisies parmi les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notament des fusions comportant une partie active fusionnée à une partie stabilisatrice (par exemple des fusions entre l'albumine ou des fragments d'albumine et le récepteur ou une partie d'un récepteur de virus (CD4, etc.)).

9. ADN recombinant selon l'une quelconque des revendications 4 à 8 caractérisé en ce qu'il fait partie d'un plasmide d'expression, qui peut être à réplication autonome ou intégratif.

10. Cellule recombinée contenant une séquence d'ADN ou un ADN recombinant selon l'une quelconque des revendications précédentes.

11. Cellule recombinée selon la revendication 10 caractérisée en ce qu'il s'agit d'une levure.

12. Cellule recombinée selon la revendication 11 caractérisée en ce qu'il s'agit d'une levure du genre Kluyveromyces.

13. Utilisation d'une séquence d'ADN selon l'une quelconque des revendications 1 à 9 pour l'expression de gènes recombinés.

14. Utilisation selon la revendication 13 pour l'expression simultanée de gènes recombinés insérés de part et d'autre du promoteur dans les 2 orientations opposées.

15. Utilisation selon l'une des revendications 13 ou 14 pour l'expression de gènes codant pour des protéines d'intérêt pharmaceutique ou agroalimentaire.

16. Procédé de production de protéines recombinantes caractérisé en ce que l'on cultive une cellule recombinée selon l'une quelconque des revendications 10 à 12 et on récupère les protéines produites.

17. Procédé selon la revendication 16 pour la production de protéines d'intérêt pharmaceutique ou agroalimentaire.

**18.** Procédé selon la revendication 17 caractérisé en ce que la protéine est préférentiellement la sérum-albumine humaine ou un de ses variants moléculaires.

```
         | 10       | 20       | 30        | 40       | 50       | 60
   1 AGATCTATCA ACGGCTACTA ATAGAAGTTC AACACCCAGA AATTGATTGT TTTGGCCTGA 60
  61 AATGTTAATG GCAGGGAATC AAGTATGAAG TATGAAGTAT GAAGTGTGAA GTGTGAATCC 120
 121 CGTAAAATCG GAGAAACATG TGGTCACCTG GTCAAGGACT TAAGGGAACA CAGCGGTTGT 180
 181 TCTCGGCATT AAGCGGTACT ATTGTCACTT TGTAATGGCA GTCCGAATCA CGTTTTATTA 240
 241 TATGGGGGGG GGGAAGGAAC GACGGTACAT AAGAATGAGG GGCTTGGTTG CGGTGTACGG 300
 301 GTTTAGTACC CCTCTCGTCC GTGCAAGCCC AAAGACAAGT TTTTGTTTCT TTTTCGTATC 360
 361 CATCCCCAGA ACAGTGCGCA GCAAGGAAAT ATTCCGTCAT GCGTTTGGAT GGTTTTTCCC 420
 421 CTATTGAGGG GCAAGTGAGG GGTAAACGAA CGGCGCCTGA AATTTTCCCG CTCAATGATA 480
 481 TGAATAATCA GTAGGATCTC CGAGTTTCGC AAGAAGCAGT AAGCAACTCC GTTTTATTGC 540
 541 GAGACAACAC TATTGTGAGA AAAGGCACTC AAAAAGCGAC CTCCGTTAAT TGATGTCAAC 600
 601 TGGCATCAAT TTGAGATACT TTGGGTCAAT TTGCAGTGTG CATTCGCATT CTTTGTGTTA 660
 661 AAATCCTTTT CCCCAGAAGT CGGAGTTTAC AGGACACAGG GTGTGGAGCA ATTGGGAGAG 720
 721 CTCCAGCAGG GCTGGAGCTC TGCCTCTGCC TCTGCCCCAG GTCCAGGTCC AAGTCCAAGT 780
 781 CCAGGTCCAG CGAGAACCGG AGTGAGAGGT GTGTGCTGTG CCTTCCACTA AACGAACCAG 840
 841 TATCCCAGGT CTCTTAAGTT TCCCAAATCT CGGCATGGTC AGGCCTCTCC ACTGTAGCAG 900
 901 CCGCAGCACA TTTTTTTTTT TTTTCTCTCT TCTAATGGAT CAAGCATCAC TACTTATCAC 960
 961 AATTTATCAC TTTTTCCAAT GATGTTGCCA TTGCCCTTGT TGGCCTTCTC GAACTAGTCC 1020
1021 GTCTTTCTGG TTTAACTTGG TGAGGGAAAT TCTTAGCACT GGACTGCGCT GTGATATGAC 1080
1081 CTGTTAAATT ATAACAAGGA GTCGTTTTTC AATTGACAAT TTCTTATCAT TGTCTCTGGG 1140
1141 ATCAATTGGT TTTTCTTCCT CTCTTTCGCT TTTCTCCCCC ACCAACAACA CAACATACAA 1200
1201 CACACGCAAT G                                                      1211
         | 10       | 20       | 30        | 40       | 50       | 60
```

Figure 1

```
        |    10       |    20       |    30        |    40       |    50       |    60
  1 GAGCTCTGCC TCTGCCTCTG CCCCAGGTCC  AGGTCCAAGT CCAAGTCCAG GTCCAGCGAG 60
 61 AACCGGAGTG AGAGGTGTGT GCTGTGCCTT  CCACTAAACG AACCAGTATC CCAGGTCTCT 120
121 TAAGTTTCCC AAATCTCGGC ATGGTCAGGC  CTCTCCACTG TAGCAGCCGC AGCACATTTT 180
181 TTTTTTTTTT CTCTCTTCTA ATGGATCAAG  CATCACTACT TATCACAATT TATCACTTTT 240
241 TCCAATGATG TTGCCATTGC CCTTGTTGGC  CTTCTCGAAC TAGTCCGTCT TTCTGGTTTA 300
301 ACTTGGTGAG GGAAATTCTT AGCACTGGAC  TGCGCTGTGA TATGACCTGT TAAATTATAA 360
361 CAAGGAGTCG TTTTTCAATT GACAATTTCT  TATCATTGTC TCTGGGATCA ATTGGTTTTT 420
421 CTTCCTCTCT TTCGCTTTTC TCCCCCACCA  ACAACACAgg atcc                  464
        |    10       |    20       |    30        |    40       |    50       |    60
```

Figure 2

```
        |   10     |   20     |   30      |   40     |   50     |   60
  1 gtcgacGCGA GACAACACTA TTGTGAGAAA  AGGCACTCAA AAAGCGACCT CCGTTAATTG 60
 61 ATGTCAACTG GCATCAATTT GAGATACTTT  GGGTCAATTT GCAGTGTGCA TTCGCATTCT 120
121 TTGTGTTAAA ATCCTTTTCC CCAGAAGTCG  GAGTTTACAG GACACAGGGT GTGGAGCAAT 180
181 TGGGAGAGCT CCAGCAGGGC TGGAGCTCTG  CCTCTGCCTC TGCCCCAGGT CCAGGTCCAA 240
241 GTCCAAGTCC AGGTCCAGCG AGAACCGGAG  TGAGAGGTGT GTGCTGTGCC TTCCACTAAA 300
301 CGAACCAGTA TCCCAGGTCT CTTAAGTTTC  CCAAATCTCG GCATGGTCAG GCCTCTCCAC 360
361 TGTAGCAGCC GCAGCACATT TTTTTTTTTT  TTCTCTCTTC TAATGGATCA AGCATCACTA 420
421 CTTATCACAA TTTATCACTT TTTCCAATGA  TGTTGCCATT GCCCTTGTTG GCCTTCTCGA 480
481 ACTAGTCCGT CTTTCTGGTT TAACTTGGTG  AGGGAAATTC TTAGCACTGG ACTGCGCTGT 540
541 GATATGACCT GTTAAATTAT AACAAGGAGT  CGTTTTTCAA TTGACAATTT CTTATCATTG 600
601 TCTCTGGGAT CAATTGGTTT TTCTTCCTCT  CTTTCGCTTT TCTCCCCCAC CAACAACACA 660
661 aagctt                                                            666
        |   10     |   20     |   30      |   40     |   50     |   60
```

Figure 3

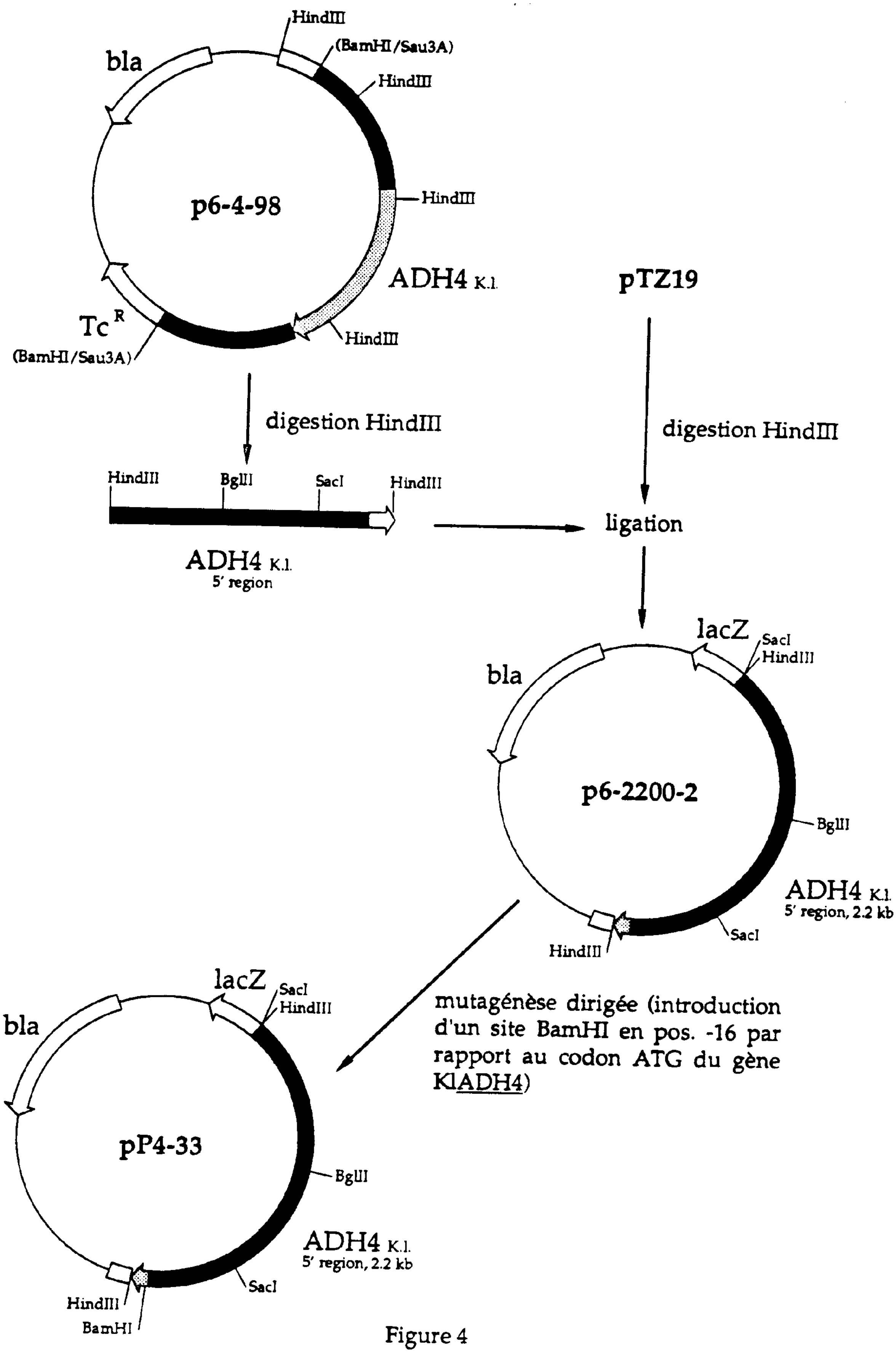
HindIII
(BamHI/Sau3A)
HindIII
bla
p6-4-98
HindIII
ADH4 K.l.
Tc R
(BamHI/Sau3A)
HindIII
pTZ19
digestion HindIII
digestion HindIII
HindIII
BglII
SacI
HindIII
ligation
ADH4 K.l.
5' region
lacZ
SacI
HindIII
bla
p6-2200-2
BglII
ADH4 K.l.
5' region, 2.2 kb
SacI
HindIII
mutagénèse dirigée (introduction d'un site BamHI en pos. -16 par rapport au codon ATG du gène KlADH4)
lacZ
SacI
HindIII
bla
pP4-33
BglII
ADH4 K.l.
5' region, 2.2 kb
SacI
HindIII
BamHI

Figure 4

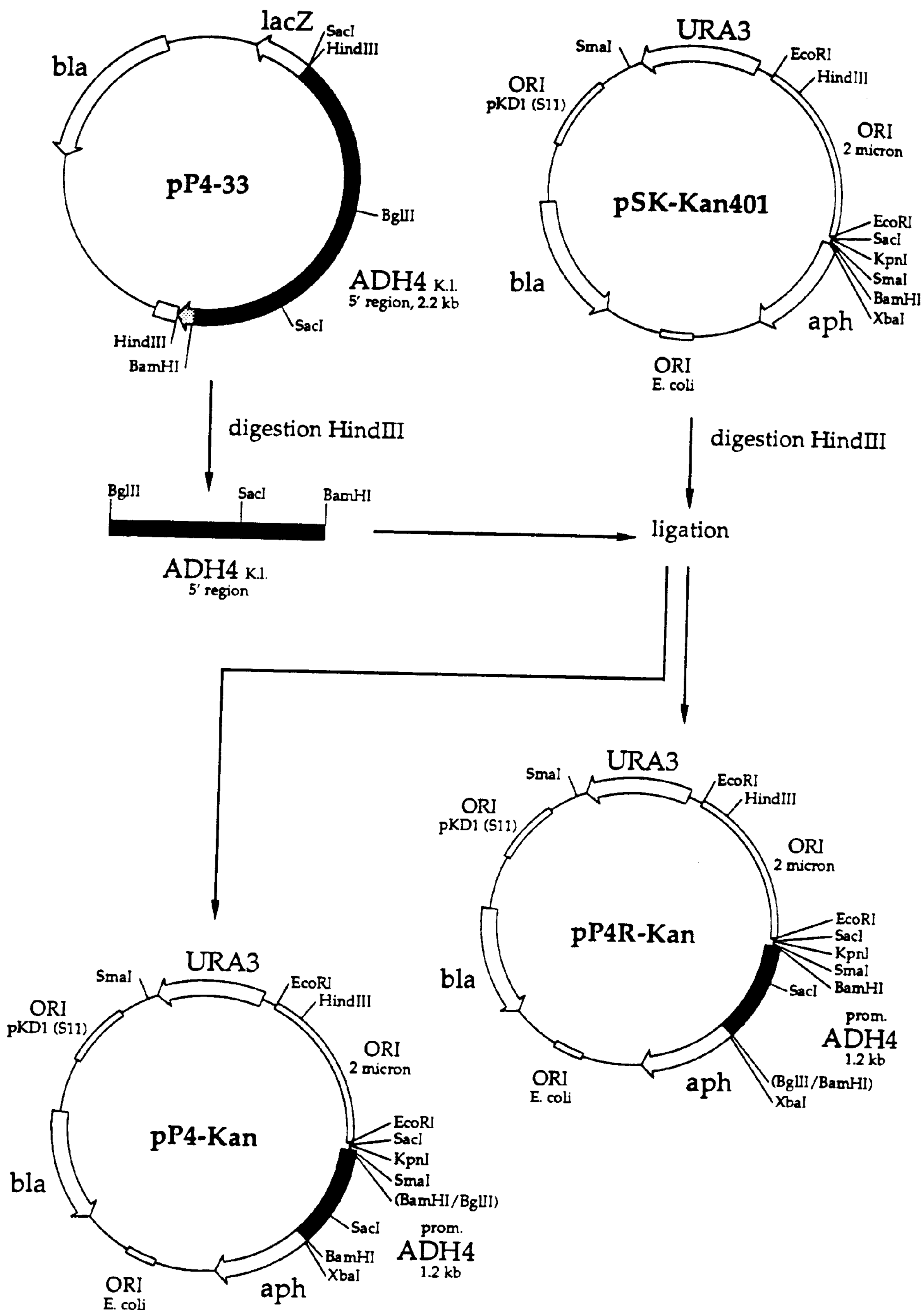
lacZ
SacI
HindIII
bla
pP4-33
BglII
ADH4 K.l.
5' region, 2.2 kb
SacI
HindIII
BamHI
URA3
SmaI
EcoRI
HindIII
ORI
pKD1 (S11)
ORI
2 micron
pSK-Kan401
EcoRI
SacI
KpnI
SmaI
BamHI
XbaI
bla
aph
ORI
E. coli
digestion HindIII
digestion HindIII
BglII
SacI
BamHI
ADH4 K.l.
5' region
ligation
URA3
SmaI
EcoRI
HindIII
ORI
pKD1 (S11)
ORI
2 micron
pP4R-Kan
EcoRI
SacI
KpnI
SmaI
BamHI
SacI
bla
prom.
ADH4
1.2 kb
ORI
E. coli
aph
(BglII/BamHI)
XbaI
URA3
SmaI
EcoRI
HindIII
ORI
pKD1 (S11)
ORI
2 micron
pP4-Kan
EcoRI
SacI
KpnI
SmaI
(BamHI/BglII)
bla
SacI
prom.
BamHI
ADH4
XbaI
1.2 kb
ORI
E. coli
aph

Figure 5

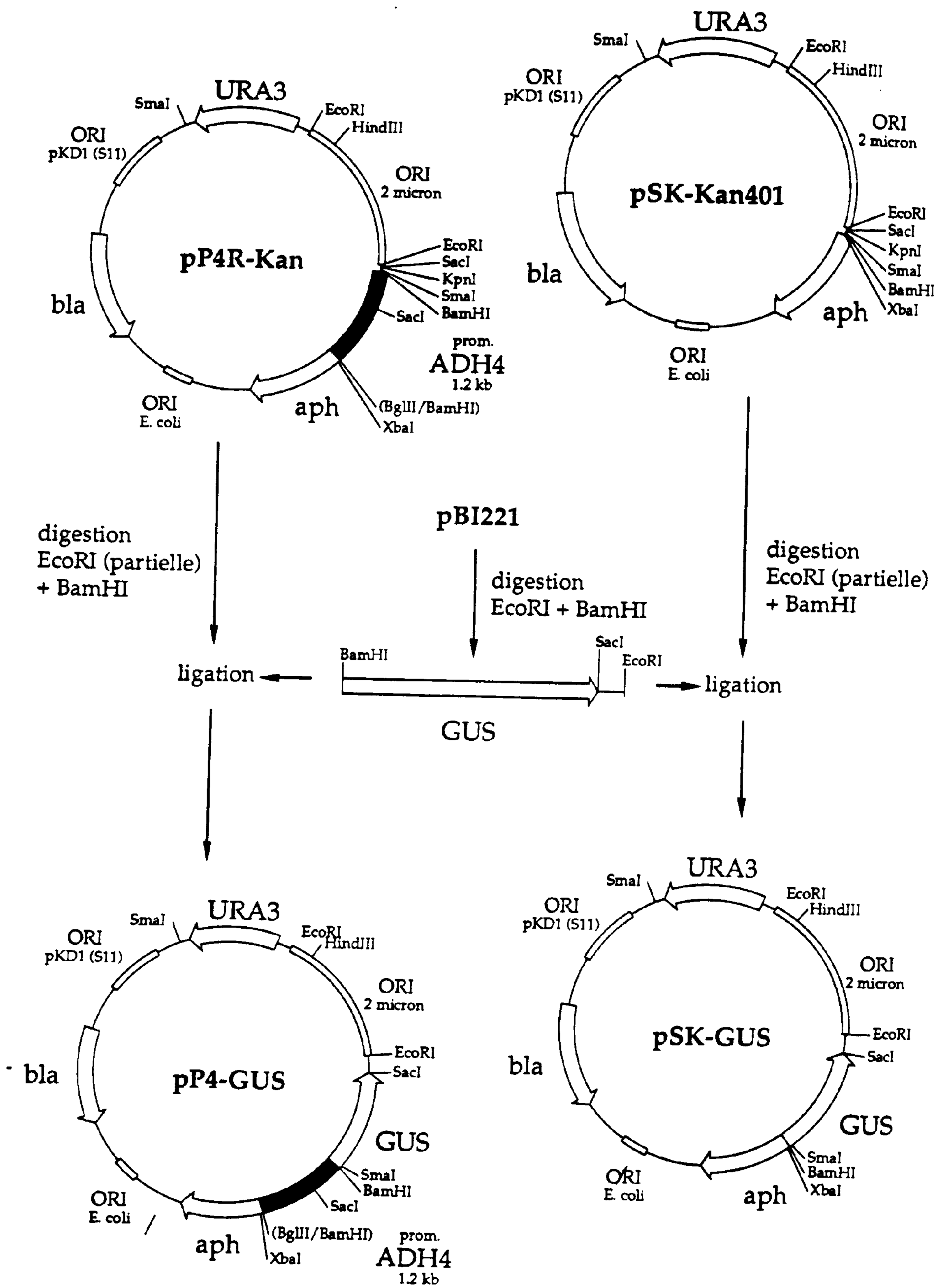
URA3
SmaI
EcoRI
HindIII
ORI
pKD1 (S11)
ORI
2 micron
pP4R-Kan
bla
EcoRI
SacI
KpnI
SmaI
BamHI
SacI
prom.
ADH4
1.2 kb
ORI
E. coli
aph
(BglII/BamHI)
XbaI
URA3
SmaI
EcoRI
HindIII
ORI
pKD1 (S11)
ORI
2 micron
pSK-Kan401
bla
EcoRI
SacI
KpnI
SmaI
BamHI
XbaI
aph
ORI
E. coli
digestion
EcoRI (partielle)
+ BamHI
pBI221
digestion
EcoRI + BamHI
digestion
EcoRI (partielle)
+ BamHI
BamHI
SacI
EcoRI
ligation
ligation
GUS
URA3
SmaI
EcoRI
HindIII
ORI
pKD1 (S11)
ORI
2 micron
EcoRI
SacI
bla
pP4-GUS
GUS
SmaI
BamHI
SacI
ORI
E. coli
aph
(BglII/BamHI)
XbaI
prom.
ADH4
1.2 kb
URA3
SmaI
EcoRI
HindIII
ORI
pKD1 (S11)
ORI
2 micron
pSK-GUS
EcoRI
SacI
bla
GUS
SmaI
BamHI
XbaI
ORI
E. coli
aph

Figure 6

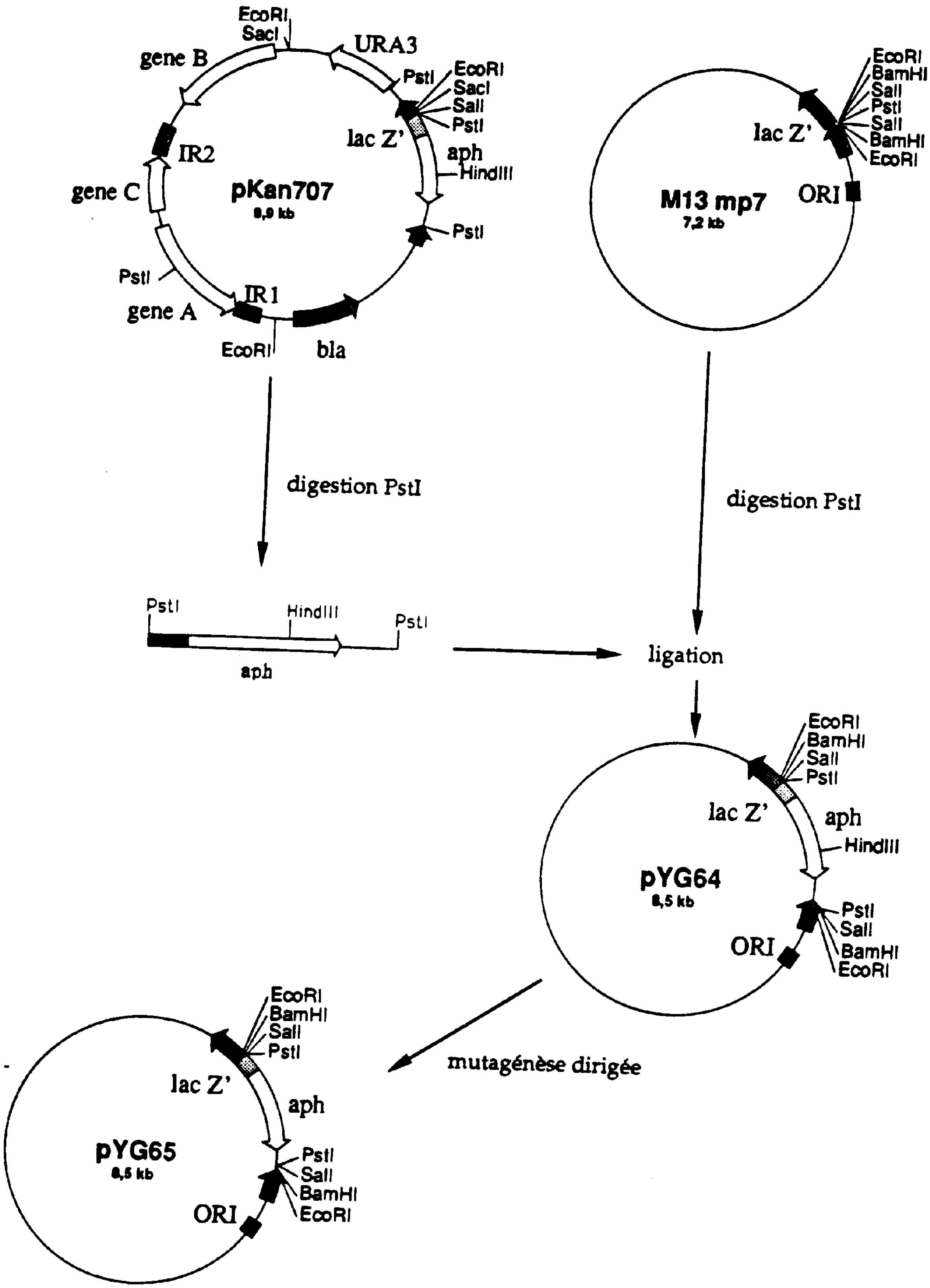
EcoRI
SacI
URA3
gene B
PstI
EcoRI
SacI
SalI
PstI
lac Z'
aph
HindIII
IR2
gene C
pKan707
8,9 kb
PstI
PstI
IR1
gene A
EcoRI
bla
EcoRI
BamHI
SalI
PstI
SalI
BamHI
EcoRI
lac Z'
M13 mp7
7,2 kb
ORI
digestion PstI
digestion PstI
PstI
HindIII
PstI
aph
ligation
EcoRI
BamHI
SalI
PstI
lac Z'
aph
HindIII
pYG64
8,5 kb
PstI
SalI
BamHI
EcoRI
ORI
mutagénèse dirigée
EcoRI
BamHI
SalI
PstI
lac Z'
aph
pYG65
8,5 kb
PstI
SalI
BamHI
EcoRI
ORI

Figure 7

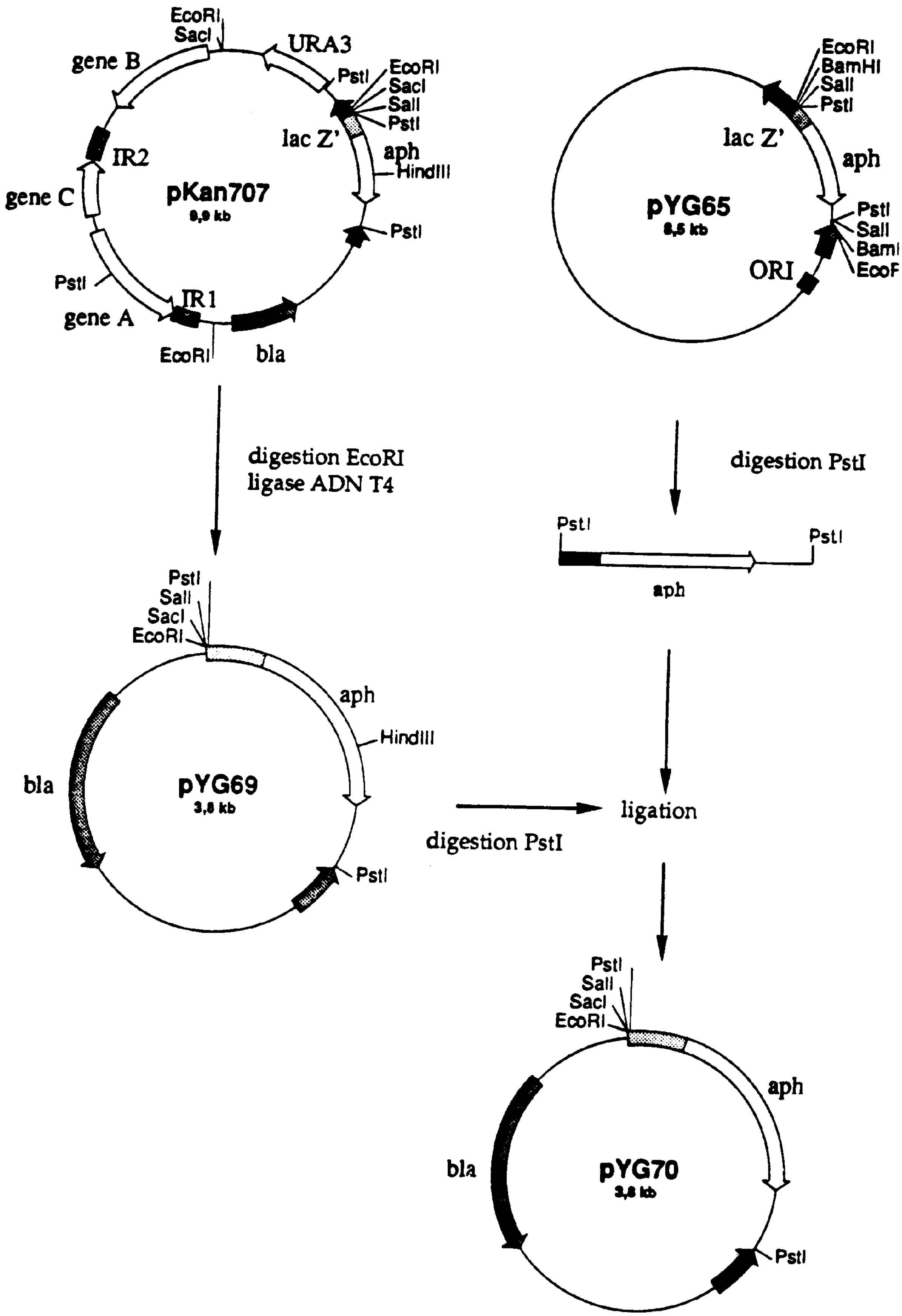
EcoRI
SacI
URA3
gene B
PstI
EcoRI
SacI
SalI
PstI
lac Z'
aph
HindIII
IR2
gene C
pKan707
8,9 kb
PstI
PstI
IR1
gene A
EcoRI
bla
EcoRI
BamHI
SalI
PstI
lac Z'
aph
pYG65
8,5 kb
PstI
SalI
BamI
EcoF
ORI
digestion EcoRI
ligase ADN T4
digestion PstI
PstI
PstI
aph
PstI
SalI
SacI
EcoRI
aph
HindIII
bla
pYG69
3,8 kb
PstI
ligation
digestion PstI
PstI
SalI
SacI
EcoRI
aph
bla
pYG70
3,8 kb
PstI

Figure 8

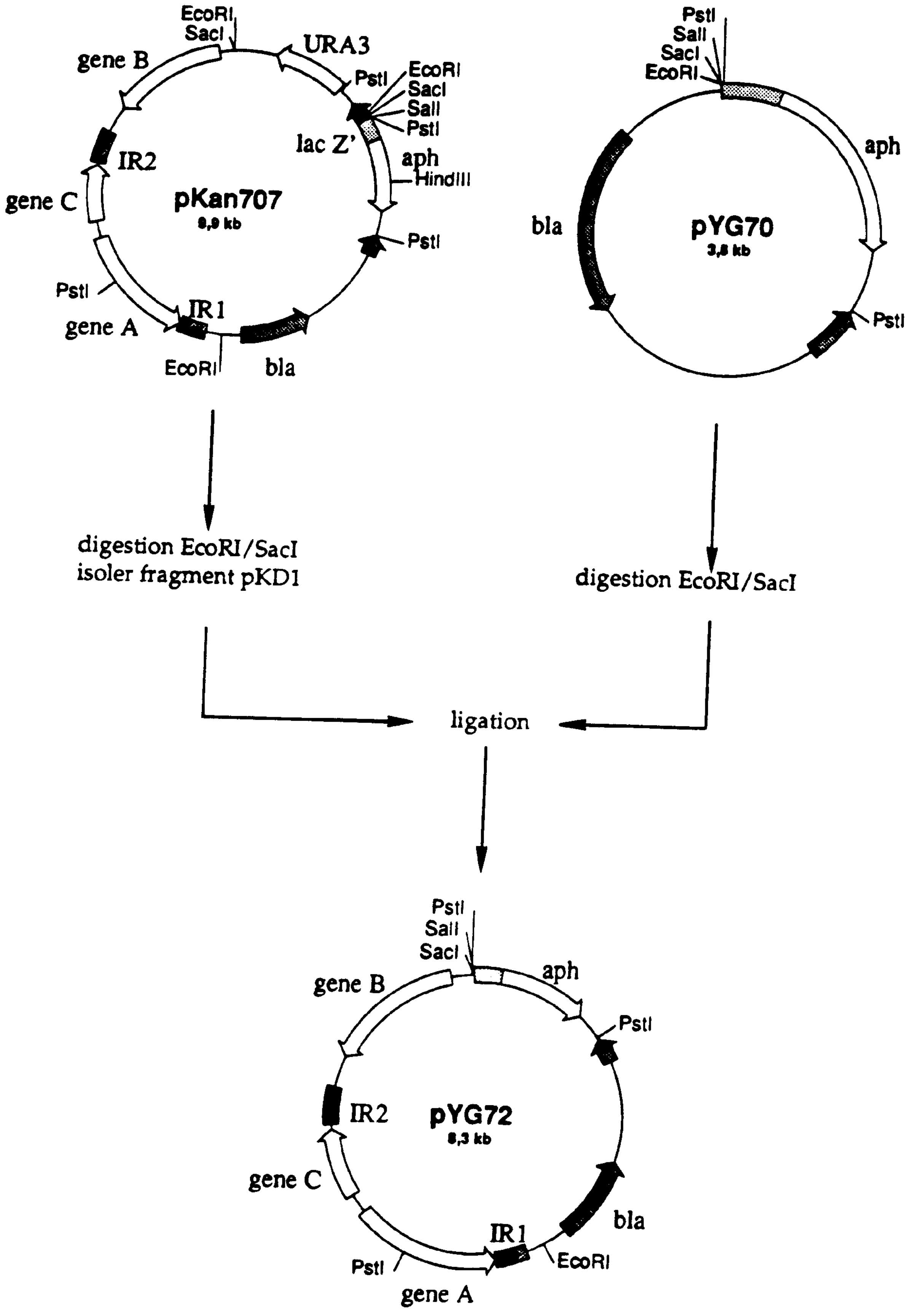
EcoRI
SacI
gene B
URA3
PstI
EcoRI
SacI
SalI
PstI
lac Z'
aph
HindIII
IR2
gene C
pKan707
8,9 kb
PstI
PstI
IR1
gene A
EcoRI
bla
PstI
SalI
SacI
EcoRI
aph
bla
pYG70
3,8 kb
PstI
digestion EcoRI/SacI
isoler fragment pKD1
digestion EcoRI/SacI
ligation
PstI
SalI
SacI
gene B
aph
PstI
IR2
pYG72
8,3 kb
gene C
bla
IR1
PstI
EcoRI
gene A

Figure 9

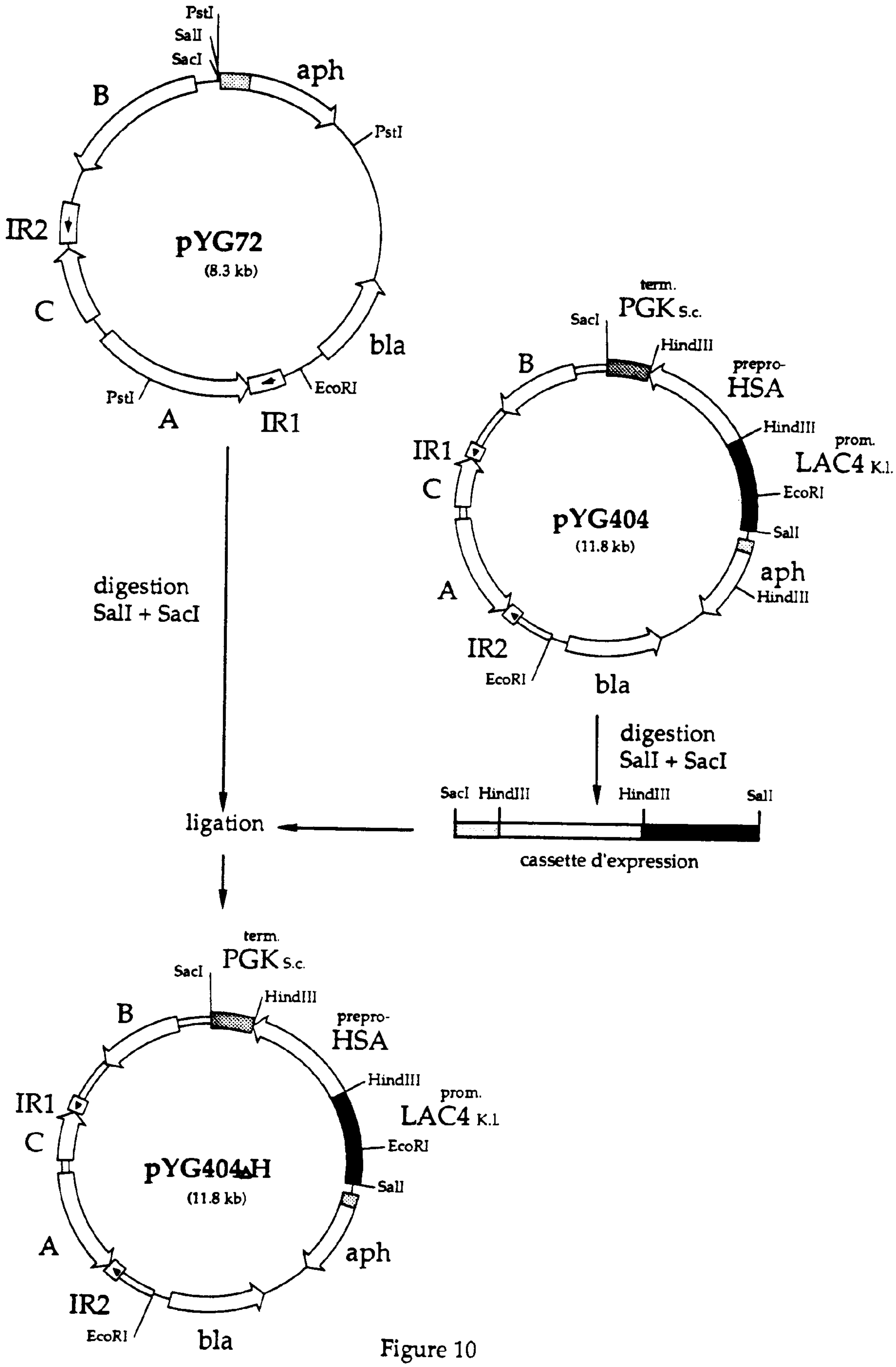
PstI
SalI
SacI
aph
PstI
B
IR2
pYG72
(8.3 kb)
C
bla
PstI
EcoRI
A
IR1
digestion
SalI + SacI
term.
SacI
PGK S.c.
HindIII
B
prepro-
HSA
HindIII
IR1
prom.
LAC4 K.l.
C
EcoRI
pYG404
(11.8 kb)
SalI
aph
A
HindIII
IR2
EcoRI
bla
digestion
SalI + SacI
SacI HindIII
HindIII
SalI
ligation
cassette d'expression
term.
SacI
PGK S.c.
HindIII
B
prepro-
HSA
HindIII
IR1
prom.
LAC4 K.l.
C
EcoRI
pYG404ΔH
(11.8 kb)
SalI
A
aph
IR2
EcoRI
bla

Figure 10

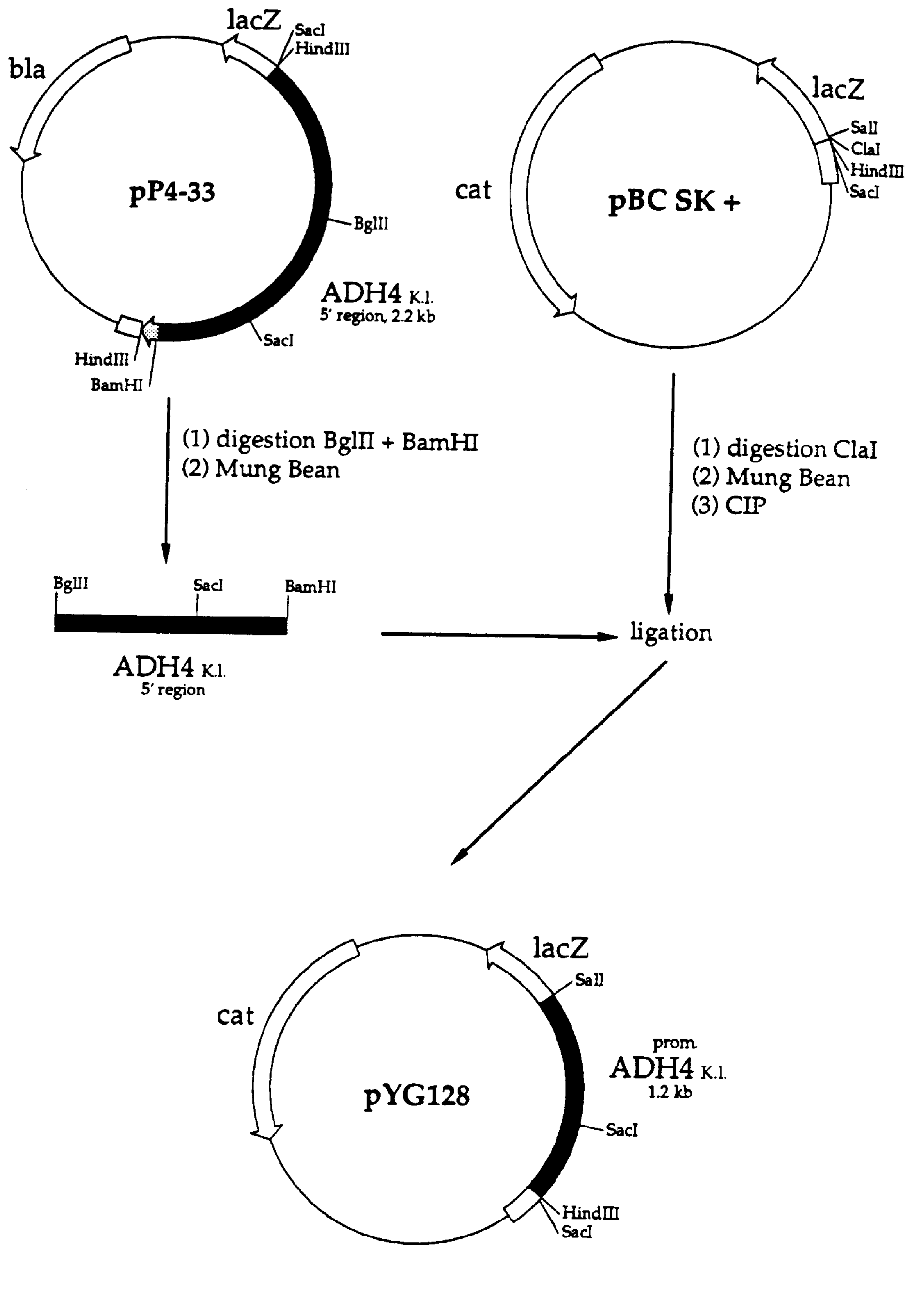
lacZ
SacI
HindIII
bla
pP4-33
BglII
ADH4 K.l.
5' region, 2.2 kb
SacI
HindIII
BamHI
cat
pBC SK +
lacZ
SalI
ClaI
HindIII
SacI
(1) digestion BglII + BamHI
(2) Mung Bean
(1) digestion ClaI
(2) Mung Bean
(3) CIP
BglII
SacI
BamHI
ADH4 K.l.
5' region
ligation
lacZ
SalI
cat
prom.
ADH4 K.l.
1.2 kb
pYG128
SacI
HindIII
SacI

Figure 11

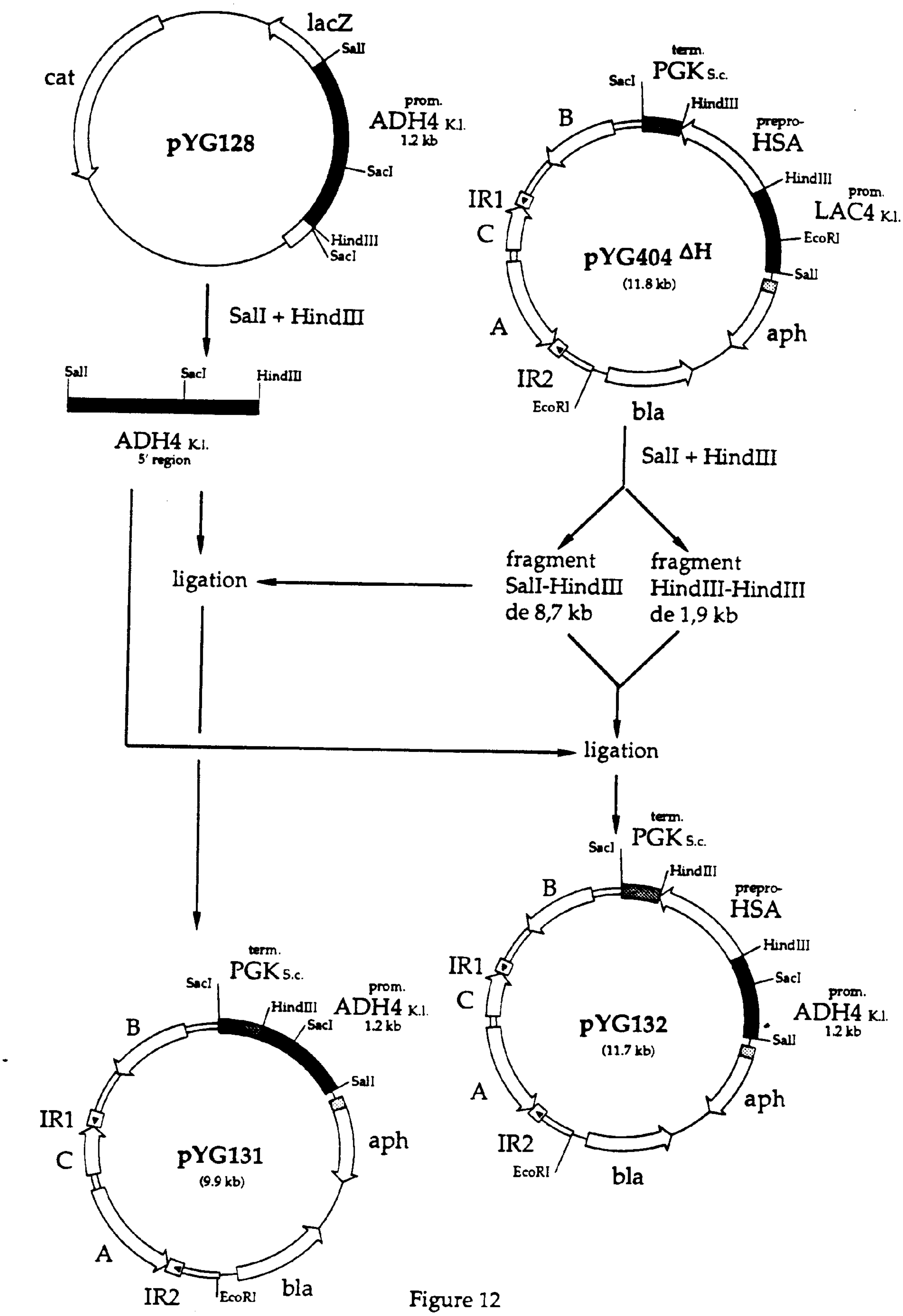

lacZ
cat
SalI
pYG128
prom.
ADH4 K.l.
1.2 kb
SacI
HindIII
SacI
SalI + HindIII
SalI
SacI
HindIII
ADH4 K.l.
5' region
term.
PGK S.c.
SacI
HindIII
B
prepro-
HSA
HindIII
prom.
LAC4 K.l.
IR1
C
EcoRI
pYG404 ΔH
(11.8 kb)
SalI
A
aph
IR2
EcoRI
bla
SalI + HindIII
fragment
SalI-HindIII
de 8,7 kb
fragment
HindIII-HindIII
de 1,9 kb
ligation
ligation
term.
PGK S.c.
SacI
HindIII
B
prepro-
HSA
HindIII
IR1
SacI
prom.
ADH4 K.l.
C
pYG132
(11.7 kb)
SalI
1.2 kb
A
aph
IR2
EcoRI
bla
term.
PGK S.c.
SacI
prom.
HindIII
ADH4 K.l.
SacI
1.2 kb
B
SalI
IR1
aph
C
pYG131
(9.9 kb)
A
IR2
EcoRI
bla


Figure 12

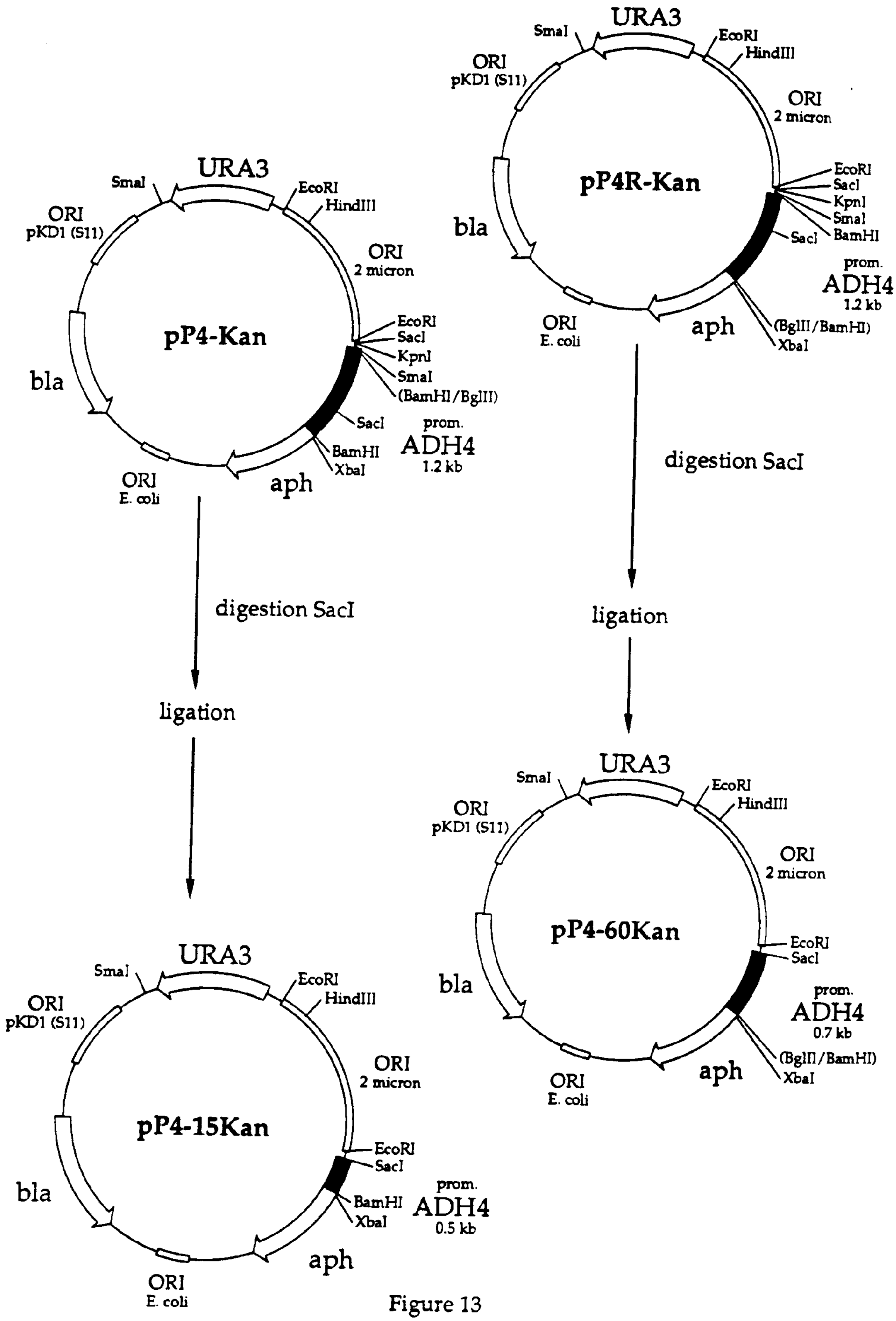
pP4-Kan
URA3
SmaI
EcoRI
HindIII
ORI
pKD1 (S11)
ORI
2 micron
EcoRI
SacI
KpnI
SmaI
(BamHI/BglII)
SacI
bla
prom.
ADH4
1.2 kb
BamHI
XbaI
ORI
E. coli
aph
digestion SacI
ligation
pP4-15Kan
URA3
SmaI
EcoRI
HindIII
ORI
pKD1 (S11)
ORI
2 micron
EcoRI
SacI
bla
prom.
ADH4
0.5 kb
BamHI
XbaI
ORI
E. coli
aph
pP4R-Kan
URA3
SmaI
EcoRI
HindIII
ORI
pKD1 (S11)
ORI
2 micron
EcoRI
SacI
KpnI
SmaI
BamHI
SacI
bla
prom.
ADH4
1.2 kb
(BglII/BamHI)
XbaI
ORI
E. coli
aph
digestion SacI
ligation
pP4-60Kan
URA3
SmaI
EcoRI
HindIII
ORI
pKD1 (S11)
ORI
2 micron
EcoRI
SacI
bla
prom.
ADH4
0.7 kb
(BglII/BamHI)
XbaI
ORI
E. coli
aph

Figure 13

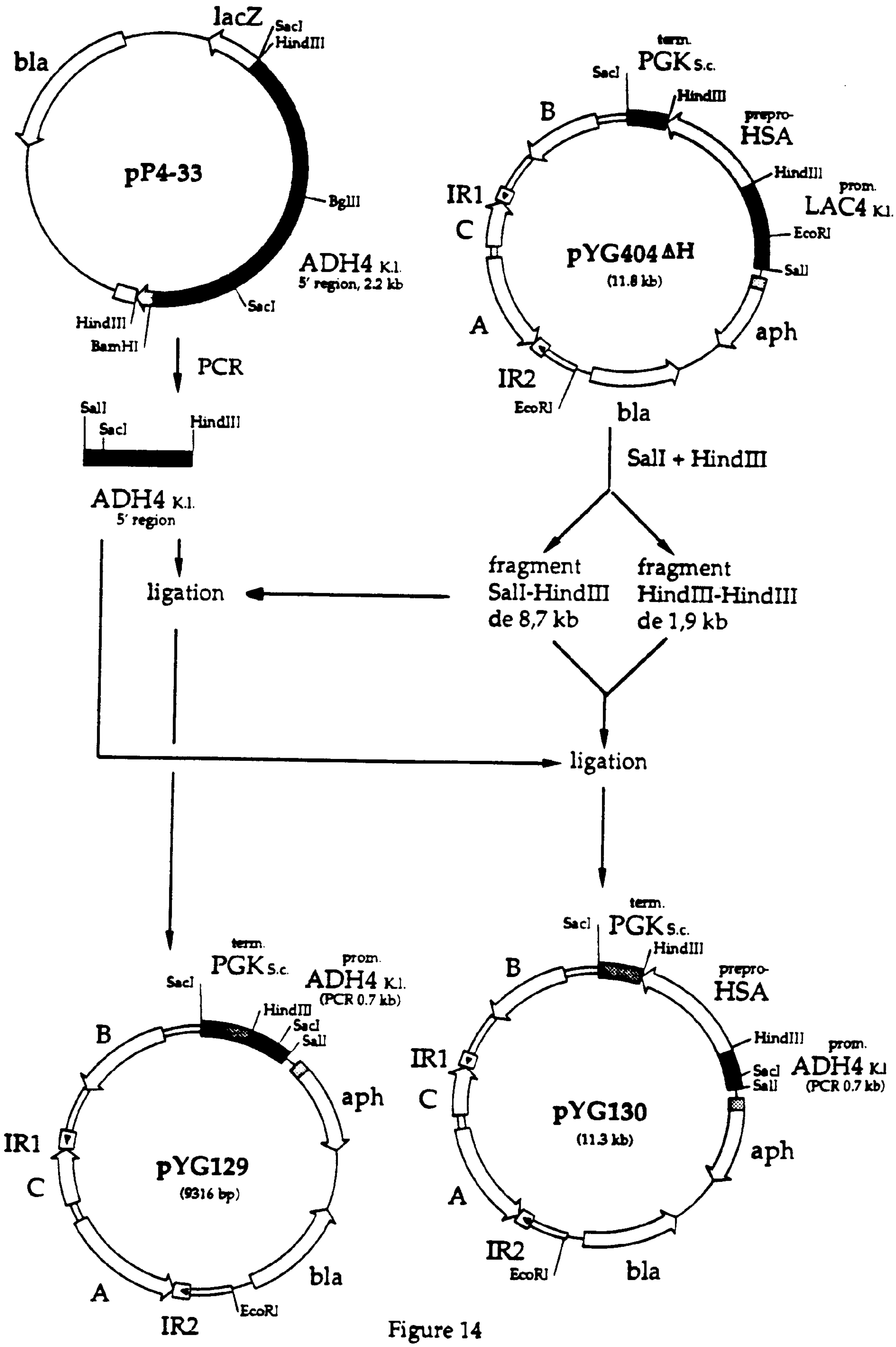
lacZ
SacI
HindIII
bla
pP4-33
BglII
ADH4 K.l.
5' region, 2.2 kb
SacI
HindIII
BamHI
PCR
SalI
SacI
HindIII
ADH4 K.l.
5' region
ligation
term.
PGK S.c.
SacI
HindIII
B
prepro-
HSA
HindIII
prom.
LAC4 K.l.
IR1
C
pYG404ΔH
(11.8 kb)
EcoRI
SalI
A
aph
IR2
EcoRI
bla
SalI + HindIII
fragment
SalI-HindIII
de 8,7 kb
fragment
HindIII-HindIII
de 1,9 kb
ligation
term.
PGK S.c.
prom.
ADH4 K.l.
(PCR 0.7 kb)
SacI
HindIII
SacI
SalI
B
aph
IR1
C
pYG129
(9316 bp)
A
bla
IR2
EcoRI
term.
PGK S.c.
SacI
HindIII
B
prepro-
HSA
HindIII
prom.
ADH4 K.l
SacI
SalI
(PCR 0.7 kb)
IR1
C
pYG130
(11.3 kb)
aph
A
IR2
EcoRI
bla

Figure 14

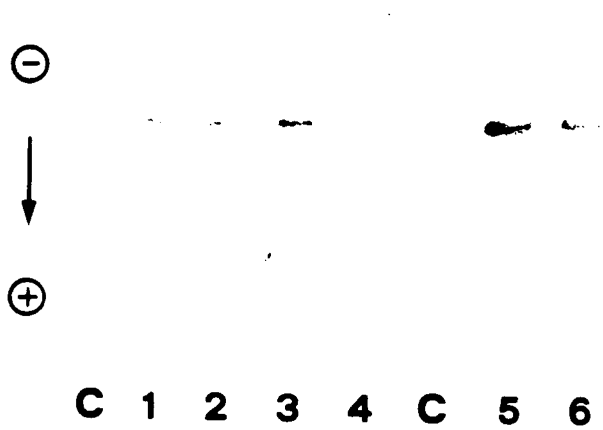


Figure 15

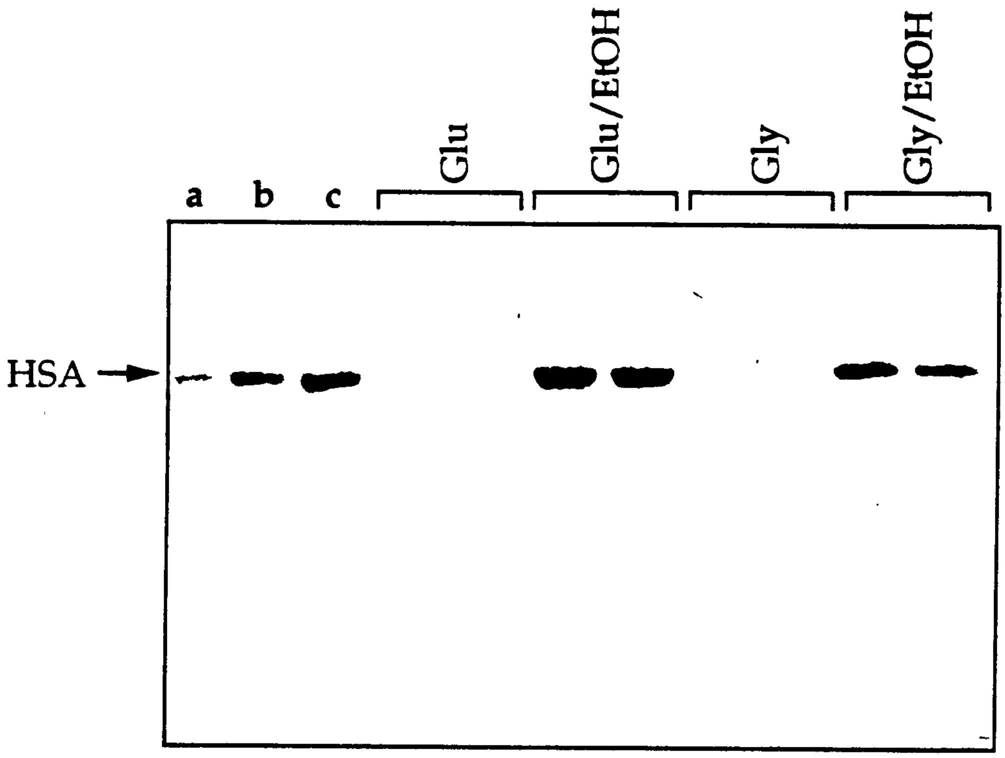


Figure 16

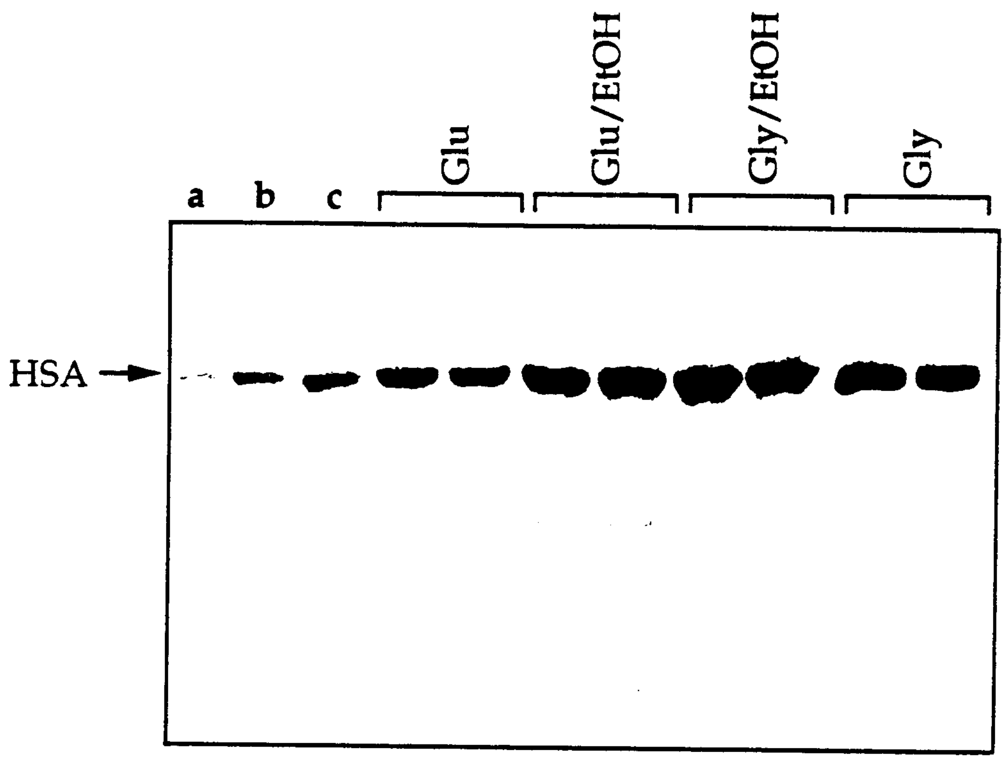


Figure 17

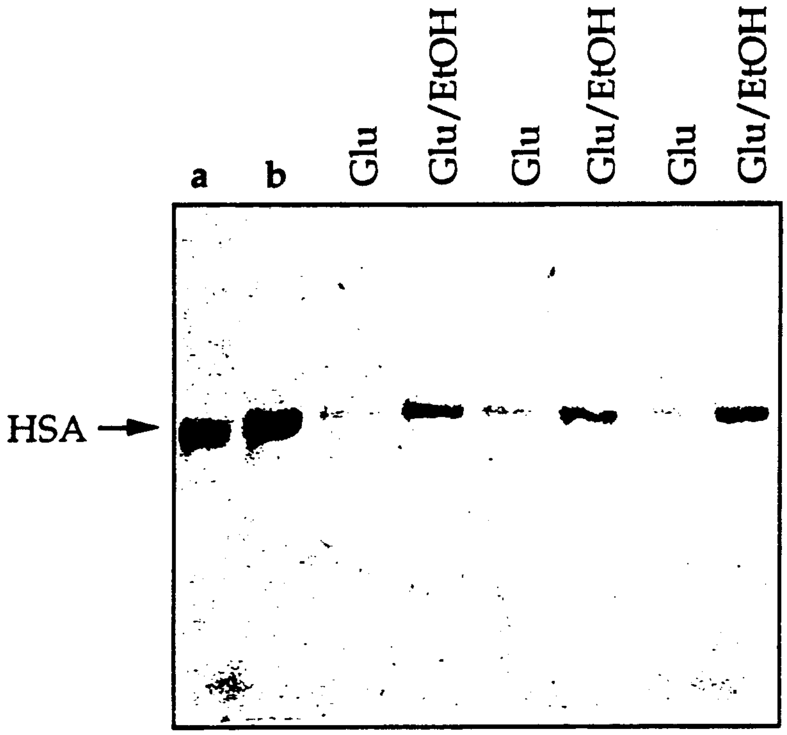


Figure 18

Office européen des brevets

# RAPPORT DE RECHERCHE EUROPEENNE

Numero de la demande
EP 92 40 2301

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | YEAST<br>vol. 7, no. 4, Juin 1991, CHICESTER, UK<br>pages 391 - 400<br>M. SALIOLA ET AL. 'Two genes encoding putative mitochondrila alcohol dehydrogenases are present in the yeast Kluyveromyces lactis'<br>--- | 1-18 | C12N15/81<br>C12N1/19<br>C12P21/02<br>//(C12N1/19,<br>C12R1:465)<br>C07K15/00 |
| A | BIOTECHNOLOGY<br>vol. 8, no. 2, Février 1990, NEW YORK US<br>pages 135 - 139<br>J.A. VAN DEN BERG ET AL. 'Kluyveromyces as a host for heterologous gene expression: expression and secretion of prochymosin'<br>* page 135, colonne 2, ligne 22 - page 135, colonne 2, ligne 41; figure 1 *<br>--- | 1-18 | |
| A | FR-A-2 635 115 (RHONE-POULENC SANTE)<br>----- | | |

| DOMAINES TECHNIQUES RECHERCHES (Int. Cl.5 ) |
|---|
| C12N<br>C07K |

Le présent rapport a été établi pour toutes les revendications

| Lieu de la recherche | Date d'achèvement de la recherche | Examinateur |
|---|---|---|
| LA HAYE | 11 DECEMBRE 1992 | VAN PUTTEN A.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

## LISTE DE SEQUENCES

(1) INFORMATION GENERALE:

(i) DEPOSANT:
(A) NOM: RHONE-POULENC RORER S.A.
(B) RUE: 20, avenue Raymond ARON
(C) VILLE: ANTONY
(E) PAYS: FRANCE
(F) CODE POSTAL: 92165

(ii) TITRE DE L' INVENTION: PROMOTEUR DE LEVURE ET SON UTILISATION.

(iii) NOMBRE DE SEQUENCES: 3

(iv) FORME LISIBLE PAR ORDINATEUR:
(A) TYPE DE SUPPORT: Tape
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(v) DONNEES DE LA DEMANDE ACTUELLE:
NUMERO DE DEPOT: EP 92402301.3

(2) INFORMATION POUR LA SEQ ID NO: 1:

(i) CARACTERISTIQUES DE LA SEQUENCE:
(A) LONGUEUR: 1211 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(vi) ORIGINE:
(A) ORGANISME: Kluyveromyces lactis

(vii) SOURCE IMMEDIATE:
(B) CLONE: 2359/152

(xi) DESCRIPTION DE LA SEQUENCE: SEQ ID NO: 1:

```
AGATCTATCA ACGGCTACTA ATAGAAGTTC AACACCCAGA AATTGATTGT TTTGGCCTGA      60
AATGTTAATG GCAGGGAATC AAGTATGAAG TATGAAGTAT GAAGTGTGAA GTGTGAATCC     120
CGTAAAATCG GAGAAACATG TGGTCACCTG GTCAAGGACT TAAGGGAACA CAGCGGTTGT     180
TCTCGGCATT AAGCGGTACT ATTGTCACTT TGTAATGGCA GTCCGAATCA CGTTTTATTA     240
TATGGGGGGG GGGAAGGAAC GACGGTACAT AAGAATGAGG GGCTTGGTTG CGGTGTACGG     300
GTTTAGTACC CCTCTCGTCC GTGCAAGCCC AAAGACAAGT TTTTGTTTCT TTTTCGTATC     360
CATCCCCAGA ACAGTGCGCA GCAAGGAAAT ATTCCGTCAT GCGTTTGGAT GGTTTTTCCC     420
CTATTGAGGG GCAAGTGAGG GGTAAACGAA CGGCGCCTGA AATTTTCCCG CTCAATGATA     480
TGAATAATCA GTAGGATCTC CGAGTTTCGC AAGAAGCAGT AAGCAACTCC GTTTTATTGC     540
GAGACAACAC TATTGTGAGA AAAGGCACTC AAAAAGCGAC CTCCGTTAAT TGATGTCAAC     600
TGGCATCAAT TTGAGATACT TTGGGTCAAT TTGCAGTGTG CATTCGCATT CTTTGTGTTA     660
AAATCCTTTT CCCCAGAAGT CGGAGTTTAC AGGACACAGG GTGTGGAGCA ATTGGGAGAG     720
CTCCAGCAGG GCTGGAGCTC TGCCTCTGCC TCTGCCCCAG GTCCAGGTCC AAGTCCAAGT     780
CCAGGTCCAG CGAGAACCGG AGTGAGAGGT GTGTGCTGTG CCTTCCACTA AACGAACCAG     840
TATCCCAGGT CTCTTAAGTT TCCCAAATCT CGGCATGGTC AGGCCTCTCC ACTGTAGCAG     900
CCGCAGCACA TTTTTTTTTT TTTTCTCTCT TCTAATGGAT CAAGCATCAC TACTTATCAC     960
AATTTATCAC TTTTTCCAAT GATGTTGCCA TTGCCCTTGT TGGCCTTCTC GAACTAGTCC    1020
GTCTTTCTGG TTTAACTTGG TGAGGGAAAT TCTTAGCACT GGACTGCGCT GTGATATGAC    1080
CTGTTAAATT ATAACAAGGA GTCGTTTTTC AATTGACAAT TTCTTATCAT TGTCTCTGGG    1140
ATCAATTGGT TTTTCTTCCT CTCTTTCGCT TTTCTCCCCC ACCAACAACA CAACATACAA    1200
CACACGCAAT G                                                         1211
```

(2) INFORMATION POUR LA SEQ ID NO: 2:

(i) CARACTERISTIQUES DE LA SEQUENCE:
(A) LONGUEUR: 464 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(xi) DESCRIPTION DE LA SEQUENCE: SEQ ID NO: 2:

```
GAGCTCTGCC TCTGCCTCTG CCCCAGGTCC AGGTCCAAGT CCAAGTCCAG GTCCAGCGAG    60
AACCGGAGTG AGAGGTGTGT GCTGTGCCTT CCACTAAACG AACCAGTATC CCAGGTCTCT   120
TAAGTTTCCC AAATCTCGGC ATGGTCAGGC CTCTCCACTG TAGCAGCCGC AGCACATTTT   180
TTTTTTTTTT CTCTCTTCTA ATGGATCAAG CATCACTACT TATCACAATT TATCACTTTT   240
TCCAATGATG TTGCCATTGC CCTTGTTGGC CTTCTCGAAC TAGTCCGTCT TTCTGGTTTA   300
ACTTGGTGAG GGAAATTCTT AGCACTGGAC TGCGCTGTGA TATGACCTGT TAAATTATAA   360
CAAGGAGTCG TTTTTCAATT GACAATTTCT TATCATTGTC TCTGGGATCA ATTGGTTTTT   420
CTTCCTCTCT TTCGCTTTTC TCCCCCACCA ACAACACAGG ATCC                    464
```

(2) INFORMATION POUR LA SEQ ID NO: 3:

(i) CARACTERISTIQUES DE LA SEQUENCE:
(A) LONGUEUR: 666 paires de bases
(B) TYPE: acide nucléique
(C) NOMBRE DE BRINS: double
(D) CONFIGURATION: linéaire

(ii) TYPE DE MOLECULE: ADNc

(iii) HYPOTHETIQUE: NON

(iii) ANTI-SENS: NON

(xi) DESCRIPTION DE LA SEQUENCE: SEQ ID NO: 3:

```
GTCGACGCGA GACAACACTA TTGTGAGAAA AGGCACTCAA AAAGCGACCT CCGTTAATTG    60
ATGTCAACTG GCATCAATTT GAGATACTTT GGGTCAATTT GCAGTGTGCA TTCGCATTCT   120
TTGTGTTAAA ATCCTTTTCC CCAGAAGTCG GAGTTTACAG GACACAGGGT GTGGAGCAAT   180
TGGGAGAGCT CCAGCAGGGC TGGAGCTCTG CCTCTGCCTC TGCCCCAGGT CCAGGTCCAA   240
GTCCAAGTCC AGGTCCAGCG AGAACCGGAG TGAGAGGTGT GTGCTGTGCC TTCCACTAAA   300
CGAACCAGTA TCCCAGGTCT CTTAAGTTTC CCAAATCTCG GCATGGTCAG GCCTCTCCAC   360
TGTAGCAGCC GCAGCACATT TTTTTTTTTT TTCTCTCTTC TAATGGATCA AGCATCACTA   420
```

```
CTTATCACAA TTTATCACTT TTTCCAATGA TGTTGCCATT GCCCTTGTTG GCCTTCTCGA   480
ACTAGTCCGT CTTTCTGGTT TAACTTGGTG AGGGAAATTC TTAGCACTGG ACTGCGCTGT   540
GATATGACCT GTTAAATTAT AACAAGGAGT CGTTTTTCAA TTGACAATTT CTTATCATTG   600
TCTCTGGGAT CAATTGGTTT TTCTTCCTCT CTTTCGCTTT TCTCCCCCAC CAACAACACA   660
AAGCTT                                                              666
```